# EUROPEAN PATENT SPECIFICATION

(11) **EP 2 916 802 B1**
(45) Date of publication and mention of the grant of the patent: **17.10.2018**
(21) Application number: 13789298.0
(22) Date of filing: 08.11.2013
(51) Int. Cl.: A61K 8/365, A61K 8/58, A61Q 5/04, A61K 8/898, A61K 8/90, A61Q 5/06, A61K 8/894

(54) **COMPOSITION COMPRISING A DICARBONYL COMPOUND AND AN AMINO SILICONE, AND PROCESS FOR STRAIGHTENING THE HAIR USING THIS COMPOSITION**
ZUSAMMENSETZUNG MIT EINER DICARBONYLVERBINDUNG UND EINEM AMINOSILIKON SOWIE HAARGLÄTTUNGSVERFAHREN UNTER VERWENDUNG DIESER ZUSAMMENSETZUNG
COMPOSITION COMPRENANT UN COMPOSÉ DICARBONYLE ET UN AMINO-SILICONE, ET PROCÉDÉ DE DÉFRISAGE DES CHEVEUX AU MOYEN DE CETTE COMPOSITION

(30) Priority: 09.11.2012 FR 1260665
(43) Date of publication of application: 16.09.2015
(73) Proprietor: L'OREAL, 75008 Paris (FR)
(72) Inventor: BIATO, Camila, CEP -22795-295 Rio de Janeiro (BR); SILVESTRE, Liliane, CEP-21235-602 Rio de Janeiro RJ (BR)
(74) Representative: Leray, Noelle
(86) International application number: PCT/EP2013/073418
(87) International publication number: WO 2014/072478

(56) References cited:
- WO-A1-2007/135299
- WO-A1-2012/105985
- WO-A2-2011/104282
- WO-A2-2012/010351
- FR-A1- 2 767 473
- US-A1- 2003 147 841

## Description

The present invention relates to a cosmetic composition, in particular a hair composition, comprising i) one or more particular dicarbonyl compounds and/or derivatives thereof and/or hydrates thereof and/or salts thereof and ii) at least one amino silicone, the composition having a pH of less than or equal to 4, and also to a process for straightening the hair using this composition.

In the hair field, consumers wish to have available compositions which make it possible to introduce a temporary change to their head of hair, while targeting good remanence of the effect produced. In general, it is desirable for the change to withstand shampooing operations for a minimum of 15 days, indeed even more, depending on the nature of the said change.

Treatments already exist for modifying the colour or the shape of the hair and also, to some extent, the texture of the hair. One of the known treatments for modifying the texture of the hair consists of the combination of heat and of a formaldehyde-comprising composition. This treatment is especially effective for imparting a better appearance to damaged hair and/or for treating long hair and curly hair.

The action of formaldehyde is associated with its ability to crosslink proteins by reaction with the nucleophilic sites thereof. The heat used may be that of an iron (flat tongs or crimping iron), the temperature of which may generally be up to 200°C or more. However, it is increasingly sought to avoid the use of such substances, which may prove to be aggressive to the hair and other keratin materials.

Patent application WO 2011/104 282 thus proposed a novel process for semi-permanently straightening the hair, which consists in applying an α-keto acid solution to the hair for 15 to 120 minutes, then drying and, finally, straightening the head of hair with an iron at a temperature of about 200°C. The α-keto acid employed is preferably glyoxylic acid. WO2012/010351, WO2012/105985, WO2007/135299 disclose as well processes for straightening the hair which consist of applying compositions based alpha-keto acid or amide thereof such as glyoxylic acid and glyoxylic amide on the hair.

However, it has been found that the use of glyoxylic acid can result in some significant limitations; in particular, its pH of action is acidic, in particular of the order of 4, which does not allow it to be used at high concentration and which may not be well tolerated, in particular when the scalp is sensitive and/or irritated. Its volatility, amplified by the use of heat (iron), can also be a problem. Furthermore, cosmetic formulations having an acidic pH may impair the hair and/or impair its colour.

It is already known practice to use glyoxylic acid esters in hair compositions, in particular in hair dye compositions, as described in document DE19859722, and in reducing compositions, as described in document DE19860239.
However, the efficacy of these compounds is not yet sufficient.

The purpose of the invention is to develop a straightening/relaxing composition which is stable over time and which makes it possible to straighten/relax and/or reduce the volume of the hair in an efficient and persistent manner while limiting damage to the hair, while at the same time retaining comfort at the time of application for the user of the composition, but also for the hairdresser who applies it.

Thus, a subject of the present invention is a cosmetic composition comprising:
i) one or more dicarbonyl compounds corresponding to formula (I) below, and/or derivatives thereof and/or hydrates thereof and/or salts thereof: 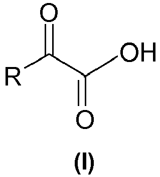 in which formula **(I):**
   **R** represents an atom or group chosen from i) hydrogen, ii) carboxyl -C(O)OH, iii) linear or branched C₁-C₆ alkyl which is optionally substituted, preferably with at least one hydroxyl -OH radical or halogen such as Br, iv) optionally substituted phenyl, v) optionally substituted benzyl, iv) and v) preferably being optionally substituted with at least one -OH or -C(O)OH radical, vi) an indolyl radical and vii) an imidazolylmethyl radical and tautomers thereof such as 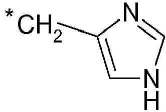 with * representing the part linked to the rest of the molecule; and
ii) at least one amino silicone, the composition having a pH of less than or equal to 4.

A subject of the invention is also a process for straightening keratin fibres, especially the hair, which comprises the application to the hair of a composition comprising:
i) one or more dicarbonyl compounds corresponding to formula (I) below, and/or derivatives thereof and/or hydrates thereof and/or salts thereof: 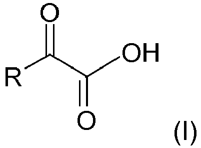 in which formula **(I):**
   **R** represents an atom or group chosen from i) hydrogen, ii) carboxyl-C(O)OH, iii) linear or branched C₁-C₆ alkyl which is optionally substituted, preferably with at least one hydroxyl -OH radical, carboxyl -C(O)-OH radical or halogen such as Br, iv) optionally substituted phenyl, v) optionally substituted benzyl, iv) and v) preferably being optionally substituted with at least one -OH or -C(O)OH radical, vi) an indolyl radical and vii) an imidazolylmethyl radical and tautomers thereof such as: 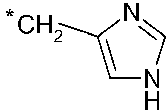 with * representing the part linked to the rest of the molecule, and
ii) at least one amino silicone,
the composition having a pH of less than or equal to 4
, followed by a straightening step using a straightening iron.

In the present invention, the dicarbonyl compounds of formula **(I)** may be in free form, but also in the form of salts or in their hydrate forms, preferably in free form or in the form of hydrates.

As "*derivatives*" of the dicarbonyl compounds of formula **(I),** mention may be made of esters of the carboxyl group(s), amides of the carboxyl group(s), and (thio)acetals and hemi(thio)acetals of the carbonyl function(s) of the compounds of formula **(I),** in free form or optionally in the form of salts or of hydrates, preferably in free form or in the form of hydrates.

The composition of the invention is stable. The composition of the invention, and the process for treating keratin fibres using ingredients i) and iii) as defined previously allow good straightening of keratin fibres while limiting damage to these keratin fibres, even when the application of the composition(s) is followed by heat treatment, in particular by means of a hair-straightening iron, and have an appreciated working quality, in particular without excessive vaporization of the composition at the time of straightening. The composition and the process for treating keratin fibres according to the invention also make it possible to limit the change in the colour of the fibres and also the problems of breaking of the said fibres such as the hair. The composition and the process of the invention will also improve the physical properties of the hair, by reducing the frizziness effect in a long-lasting manner.

In the text hereinbelow, the term "*at least one*" is equivalent to the term "*one or more*"*.*

Preferably, the composition according to the invention comprises neither a colouring agent nor a reducing agent.

According to the present invention, the term "*colouring agents*" means agents for colouring keratin fibres such as direct dyes, pigments or oxidation dye precursors (bases and couplers). If they are present, their content does not exceed 0.001% by weight relative to the total weight of the composition. Indeed, at such a content, only the composition would be dyed, i.e. no dyeing effect would be observed on the keratin fibres.

It is recalled that oxidation dye precursors, oxidation bases and couplers are colourless or sparingly coloured compounds, which, via a condensation reaction in the presence of an oxidizing agent, give a coloured species. With regard to direct dyes, these compounds are coloured and have a certain affinity for keratin fibres.

According to the present invention, the term "*reducing agent*" means an agent that is capable of reducing the disulfide bonds of the hair, such as compounds chosen from thiols, alkali metal sulfites, hydrides and phosphines.

Preferably, the dicarbonyl compound(s) corresponding to formula **(I)** and/or derivatives thereof and/or hydrates thereof and/or salts thereof are chosen from the dicarbonyl derivatives corresponding to formula **(I)** in which **R** represents i) a hydrogen atom or ii) a linear or branched C₁-C₆ alkyl group optionally substituted with a carboxyl group.

More preferably, they are chosen from glyoxylic acid and pyruvic acid, a derivative thereof, salts thereof and hydrates thereof and more preferentially from glyoxylic acid, derivatives thereof and the hydrate form thereof.

As glyoxylic acid derivatives, mention may be made of glyoxylic acid esters, glyoxylic acid amides, glyoxylic acid (thio)acetals and hemi(thio)acetals, and glyoxylic acid ester (thio)acetals and hemi(thio)acetals.

The esters and amides may be synthesized via conventional esterification or amidation processes from the corresponding acids well known to those skilled in the art.

Preferentially, the dicarbonyl compound(s) of formula **(I)** of the invention are chosen from glyoxylic acid and derivatives thereof and the hydrate forms of these compounds.

Mention may first of all be made of glyoxylic acid and also the hydrate form thereof (HO)₂CH-C(O)-OH, such as, for example, the glyoxylic acid in aqueous solution at 50% sold by the company Merck.

The glyoxylic acid esters are, for example, obtained from glyoxylic acid and a mono- or polyalcohol.

The term "*mono- or polyalcohol*" means an organic compound comprising a hydroxyl group (monoalcohol) or at least two hydroxyl groups (polyalcohol or polyol), it being possible for the said hydroxylated organic compound to be aliphatic, acyclic, linear or branched, or (hetero)cyclic, such as sugars (mono- or polysaccharides) or sugar alcohols.

More particularly, the polyalcohol comprises from 2 to 100 hydroxyl groups, preferentially from 2 to 20 hydroxyl groups, even more preferentially from 2 to 10 hydroxyl groups and better still 2 or 3 hydroxyl groups.

Preferably, the mono- or polyalcohol is chosen from methanol, ethanol, propanol, isopropanol, butanol, hexanol, ethylene glycol, glycerol, dihydroxyacetone, glucose, sorbitol and menthol.

Esters that may in particular be mentioned include methyl glyoxylate, ethyl glyoxylate, glyceryl glyoxylate, dihydroxyacetone glyoxylate, glyceryl diglyoxylate or triglyoxylate, sorbitol mono-, di- or triglyoxylate, glucose mono-, di- or triglyoxylate, menthyl glyoxylate, and the acetals, hemiacetals and hydrates thereof.

The glyoxylic acid amides are obtained, for example, from glyoxylic acid and an organic mono- or polyamine.

The term "*mono- or polyamine*" means an organic compound comprising an amino (monoamine) group or at least two (and preferably from 2 to 100, better still from 2 to 20) amino groups, it being possible for the said organic compound to be aliphatic, acyclic, linear or branched or (hetero)cyclic.

The term "*amino*" group means a primary amine group -NH₂ or a secondary amine group >NH.

Preferably, the mono- or polyamine is aliphatic.

This amine is preferably chosen from methylamine, ethylamine, propylamine, isopropylamine, butylamine, hexylamine, monoethanolamine, monopropanolamine, propane-1,2,3-triamine and diaminoacetone.

Mention may be made in particular of glyoxylic acid *N*-β-hydroxyethylamide and glyoxylic acid *N*-γ-hydroxypropylamide and the acetals, hemiacetals and hydrates thereof.

The glyoxylic acid (thio)acetals and hemi(thio)acetals may be obtained, for example, from the reaction of alcohols, for the acetals or hemiacetals, or of thiols, for the thioacetals or hemithioacetals, with blocked forms of glyoxylic acid, followed by hydrolysis. The alcohols may be the same as those mentioned for the esters. The thiols may be equivalents (referred to as mono- or polythiols) to the mono- or polyalcohols mentioned above, except for the fact that the hydroxyl function(s) of the said mono- or polyalcohols are replaced with one or more thiol functions SH of the mono- or polythiols. The acetals or thioacetals may also be cyclic (thio)acetals.

Mention may in particular be made of dimethoxyacetic acid, diethoxyacetic acid, 1,3-dioxane-2-carboxylic acid and 1,3-dioxolane-2-carboxylic acid.

The salts may be salts derived from the interaction of the compounds of formula **(I)** with acids or bases, it being possible for the acids or bases to be of organic or mineral nature.

Preferably, the salts are salts derived from the interaction of the compounds of formula **(I)** with bases. Mention will be made in particular of the salts of alkali metals or alkaline-earth metals and in particular the sodium salts.

According to one embodiment, the composition of the invention comprises from 0.1% to 20% of one or more dicarbonyl compounds corresponding to formula **(I)** and/or derivatives thereof and/or hydrates thereof and/or salts thereof, preferably at least 3% by weight and preferentially from 3% to 10% by weight relative to the total weight of the composition.

The composition according to the invention also comprises one or more amino silicones.

The term "*amino silicone*" means any silicone comprising at least one primary, secondary or tertiary amine function or a quaternary ammonium group. Preferably, the amino silicone(s) used in the cosmetic composition according to the present invention comprise in their structure at least 4 silicon atoms.

In the text hereinbelow, the term "silicone" is intended to denote, in accordance with what is generally accepted, any organosilicon polymer or oligomer of linear or cyclic, branched or crosslinked structure, of variable molecular weight, obtained by polymerization and/or polycondensation of suitably functionalized silanes, and consisting essentially of a repetition of main units in which the silicon atoms are linked together via oxygen atoms (siloxane bond -Si-O-Si-), optionally substituted hydrocarbon-based radicals being directly linked via a carbon atom to the said silicon atoms. The hydrocarbon-based groups that are the most common are alkyl groups, especially of C₁-C₁₀, and in particular methyl, fluoroalkyl groups, the alkyl part of which is of C₁-C₁₀, and aryl groups and in particular phenyl.

The amino silicones used in the composition according to the present invention may be chosen from the silicones ***(a)*** to ***(e)*** below:
***(a)*** the compounds corresponding to formula **(Ia)** below:

   (R₁)ₐ(T)₃₋ₐ-Si[OSi(T)₂]ₙ-[OSi(T)_{b}(R₁)_{2-b}]ₘ-OSi(T)₃₋ₐ-(R₁)ₐ **(Ia)**

   in which formula **(Ia):**
   - T represents a hydrogen atom or a phenyl, hydroxyl (-OH) or C₁-C₈ alkyl group, and preferably methyl, or a C₁-C₈ alkoxy, preferably methoxy,
   - a denotes the number 0 or an integer from 1 to 3, and preferably 0,
   - b denotes 0 or 1, and in particular 1,
   - **m** and **n** are integers such that the sum (n + m) can range especially from 1 to 2000 and in particular from 50 to 150, it being possible for n to denote a number from 0 to 1999 and in particular from 49 to 149, and for m to denote a number from 1 to 2000 and in particular from 1 to 10,
   - **R₁** is a monovalent group of formula -C_{q}H_{2q}L in which **q** is an integer from 2 to 8 and L is an optionally quaternized amino group chosen from the following groups:

      -N(R₂)-CH₂-CH₂-N(R₂)₂,

      -N(R₂)₂,

      -N⁺(R₂)₃Q⁻,

      -N⁺(R₂)(H)₂Q⁻,

      -N⁺(R₂)₂HQ⁻,

      -N(R₂)-CH₂-CH₂-N⁺(R₂)(H)₂Q⁻,

      in which R₂ may denote a hydrogen atom, a phenyl group, a benzyl group or a saturated monovalent hydrocarbon-based group, for example a C₁-C₂₀ alkyl group, and Q⁻ represents an anionic counterion such as a halide ion, for instance fluoride, chloride, bromide or iodide.
      In particular, the amino silicones corresponding to the definition of formula (Ia) are chosen from the compounds corresponding to formula **(IIa)** below: 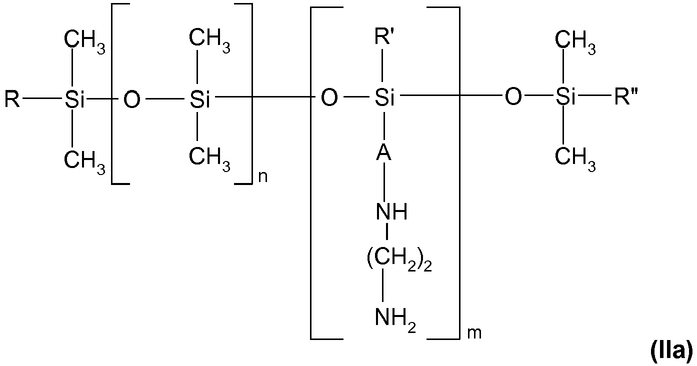 in which formula **(IIa):**
      - **R, R'** and **R",** which may be identical or different, denote a C₁-C₄ alkyl group, preferably methyl, a C₁-C₄ alkoxy group, preferably methoxy, or hydroxyl,
      - **A** represents a linear or branched C₃-C₈ and preferably C₃-C₆ alkylene group,
      - **m** and **n** are integers that are dependent on the molecular weight and whose sum is between 1 and 2000 inclusive.

   According to a particular embodiment of the invention, the amino silicones are of formula **(IIa)** with **R, R'** and **R",** which may be identical or different, each representing a C₁-C₄ alkyl or hydroxyl group, **A** represents a C₃ alkylene group and **m** and **n** are such that the weight-average molecular mass of the compound is between 5000 and 500 000 inclusive. Compounds of this type are referred to in the CTFA dictionary as "amodimethicones".
   According to another particular embodiment of the invention, the amino silicones are of formula **(IIa)** with **R, R'** and **R",** which may be identical or different, each representing a C₁-C₄ alkoxy or hydroxyl group, at least one of the groups **R** or **R"** is an alkoxy group and **A** represents a C₃ alkylene group. The hydroxyl/alkoxy mole ratio is preferably between 0.2/1 and 0.4/1 inclusive and advantageously equal to 0.3/1. Moreover, m and n are such that the weight-average molecular mass of the compound is between 2000 and 10⁶ inclusive. More particularly, n is between 0 and 999 inclusive and m is between 1 and 1000 inclusive, the sum of n and m being between 1 and 1000 inclusive.
   In this category of compounds, mention may be made, inter alia, of the product Belsil®ADM 652 sold by the company Wacker.
   According to yet another particular embodiment of the invention, the amino silicones are of formula **(IIa)** with **R** and **R",** which are different, each representing a C₁-C₄ alkoxy or hydroxyl group, at least one of the groups **R** or **R"** being an alkoxy group, **R'** representing a methyl group and **A** representing a C₃ alkylene group. The hydroxyl/alkoxy mole ratio is preferably between 1/0.8 and 1/1.1 inclusive and advantageously is equal to 1/0.95. Moreover, **m** and **n** are such that the weight-average molecular mass of the compound is between 2000 and 200 000 inclusive. More particularly, **n** is between 0 and 999 inclusive and **m** is between 1 and 1000 inclusive, the sum of n and m being between 1 and 1000.
   More particularly, mention may be made of the product Fluid WR® 1300 sold by the company Wacker.
   It should be noted that the molecular mass of these silicones is determined by gel permeation chromatography (room temperature, polystyrene standard; µ styragem columns, THF eluent, flow rate of 1 mm/m, 200 µl of a solution containing 0.5% by weight of silicone in THF are injected, and detection is performed by refractometry and UV-metry).
   According to a particularly advantageous embodiment of the invention, the amino silicone is of formula **(Ia)** and in particular the polymer known in the CTFA dictionary as "trimethylsilyl amodimethicone", corresponding to formula **(IIIa)** below: 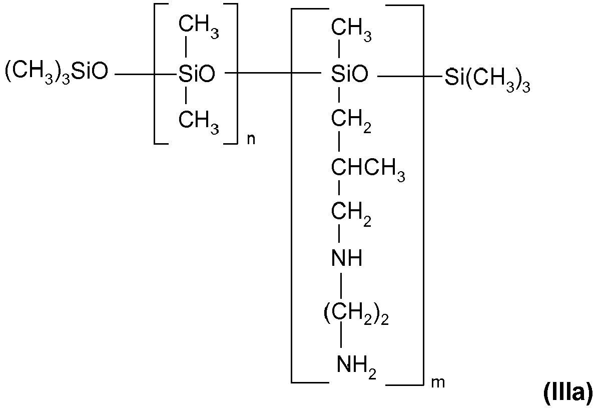 in which formula **(IIIa) n** and m have the meanings given above in accordance with formula (Ia). Such compounds are described, for example, in patent EP 95238. A compound of formula (Ia) is sold, for example, under the name Q2-8220 by the company OSI.
***(b)*** the compounds corresponding to formula **(Ib)** below: 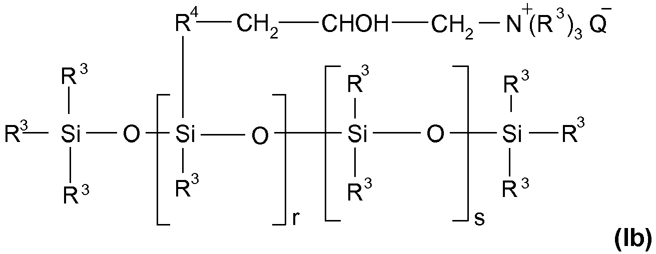 in which formula **(Ib):**
   - **R³** represents a monovalent C₁-C₁₈ hydrocarbon-based group, and in particular a C₁-C₁₈ alkyl or C₂-C₁₈ alkenyl group, for example methyl,
   - **R⁴** represents a divalent hydrocarbon-based group, in particular a C₁-C₁₈ alkylene group or a divalent C₁-C₁₈, for example C₁-C₈, alkylenoxy group,
   - **Q⁻** represents an anionic counterion such as that chosen from halide ions, especially chloride,
   - **r** represents a mean statistical value from 2 to 20 and in particular from 2 to 8,
   - **s** represents a mean statistical value between 20 and 200 inclusive and in particular between 20 and 50 inclusive.
   Such compounds are described more particularly in patent US 4 185 087. A compound falling within this class is the product sold by the company Union Carbide under the name Ucar Silicone ALE 56.
***(c)*** the quaternary ammonium silicones, especially of formula **(Ic):** 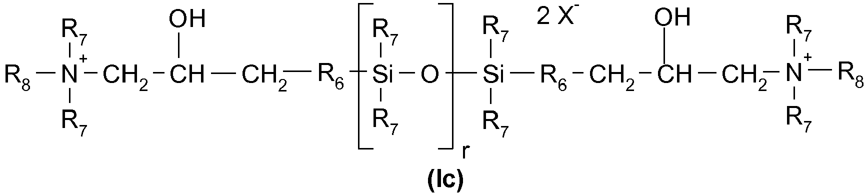 in which formula **(Ic):**
   - **R₆** represents a divalent hydrocarbon-based group, especially a C₁-C₁₈ alkylene group or a divalent C₁-C₁₈, for example C₁-C₈, alkylenoxy group linked to the Si atom via an Si-C bond,
   - **R₇,** which may be identical or different, represent a monovalent hydrocarbon-based group containing from 1 to 18 carbon atoms, and in particular a C₁-C₁₈ alkyl group, a C₂-C₁₈ alkenyl group or a ring comprising 5 or 6 carbon atoms, for example methyl,
   - **R₈,** which may be identical or different, each represent a hydrogen atom, a monovalent hydrocarbon-based group containing from 1 to 18 carbon atoms, and in particular a C₁-C₁₈ alkyl group, a C₂-C₁₈ alkenyl group or a group -R₆-N(H)-C(O)-R₇ with R₆ and R₇ as defined previously,
   - **X**⁻**,** which may be identical or different, represents an anionic counterion such as a halide ion, especially chloride, or an anionic counterion derived from an organic acid such as (C₁-C₆)alkylcarboxylate, for instance acetate, mesylate, etc.,
   - r represents a mean statistical value between 2 and 200 inclusive and in particular between 5 and 100. These silicones are described, for example, in patent application EP-A-0 530 974.
***(d)*** the amino silicones of formula **(Id):** 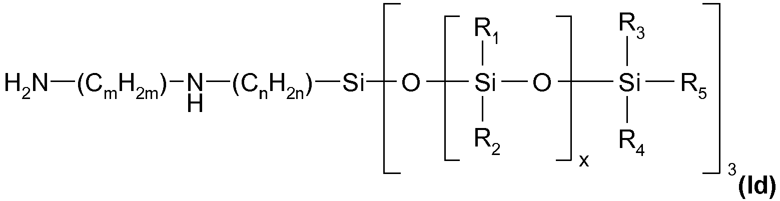 in which formula **(Id):**
   - **R₁, R₂, R₃** and **R₄,** which may be identical or different, each denote a C₁-C₄ alkyl group or an aryl group such as phenyl,
   - **R₅** denotes a C₁-C₄ alkyl group or a hydroxyl group,
   - **n** and **m,** which may be identical or different, represent an integer between 1 and 5 inclusive, and
   - **x** is such that the amine number is between 0.01 and 1 meq/g.
***(e)*** the amino silicones containing polyalkoxylene groups of formula **(Ie):** 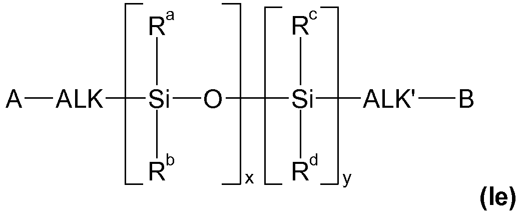 in which formula **(Ie):**
   - **R^{a}, R^{b}, R^{c}** and **R^{d},** which may be identical or different, represent a hydroxyl or linear or branched (C₁-C₁₀)alkyl group, and preferably **R^{a}, R^{b}, R^{c}** and **R^{d}** represent a (C₁-C₆)alkyl group, which is more particularly linear, such as methyl;
   - **ALK** and **ALK',** which may be identical or different, represent a linear or branched (C₁-C₁₀)alkylene group, which is preferably linear, such as propylene;
   - **A** and **B,** which may be identical or different, represent an aminopolyalkoxy group below:

      R^{e}R^{f}N-[ALK"-O]_{z}[ALK"'-O]_{w}-ALKₐ-N(R^{g})-[ALK_{b}-O]_{q}-*

      with:
      - * representing the point of attachment of the radical to the rest of the molecule via ALK or ALK';
      - **R^{e}, R^{f}** and **R^{g},** which may be identical or different, representing a hydrogen atom or a linear or branched (C₁-C₁₀)alkyl group, and preferably **R^{e}, R^{f}** and **R^{g}** represent a hydrogen atom;
      - **ALK"** and **ALK"',** which may be identical or different, represent a linear or branched (C₁-C₁₀)alkylene group, preferably of C₂ or C₃; more particularly, ALK" represents a divalent group -CH₂-CH(CH₃)- and ALK"' represents an ethylene group;
      - **ALKₐ** and **ALK_{b},** which may be identical or different, represent a linear or branched (C₁-C₁₀)alkylene group, which is optionally substituted preferably with a hydroxyl group, and is preferably of C₂ or C₃; more particularly, ALKₐ represents an ethylene or propylene group or a divalent group -CH₂-CH(CH₃)- and ALK_{b} represents a divalent group -CH₂-CH(OH)-CH₂-;
      - **q,** which may be identical or different, represent 0 or 1, preferably 1;
      - **w,** which may be identical or different, represent an integer, preferably the sum of the w values (w of A + w of B) having a mean value inclusively between 10 and 100, more particularly inclusively between 20 and 60 and more preferentially between 30 and 50, such as 40-41;
      - **z,** which may be identical or different, represent an integer, preferably the sum of the z values (z of A + z of B) having a mean value inclusively between 1 and 20, more particularly inclusively between 1 and 10 and more preferentially between 2 and 5, such as 3.
***(I'e)*** the amino silicones containing polyalkoxylene groups consisting of polysiloxane block(s) and of polyalkoxylene block(s) comprising at least one amine group, in particular:
   - those of formula **(I'e):** 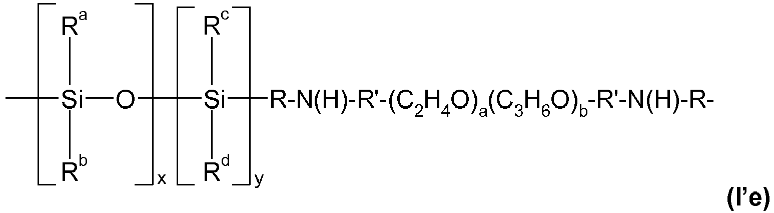 in which formula **(I'e):**
      - **R^{a}, R^{b}, R^{c}** and **R^{d},** which may be identical or different, represent a hydroxyl or linear or branched (C₁-C₁₀)alkyl group, and preferably **R^{a}, R^{b}, R^{c}** and **R^{d}** represent a (C₁-C₄)alkyl group, which is more particularly linear, such as methyl;
      - **R** and **R"**, which may be identical or different, represent a linear or branched, optionally hydroxylated C₂-C₆ alkylene radical, optionally interrupted with an oxygen atom;
      - **a** and **b,** which may be identical or different, represent a number ranging from 0 to 100;
      - **R'** and **R"',** which may be identical or different, represent a hydrogen atom or a C₁-C₄ alkyl radical such as a methyl radical;
      - **x** denotes an integer ranging from 1 to 500 and **y** denotes an integer ranging from 1 to 10;

      According to a particularly advantageous embodiment of the invention, the amino silicone is bisamino PEG/PPG-41-3 aminoethyl PG-propyl dimethicone of formula **(I"e)**: 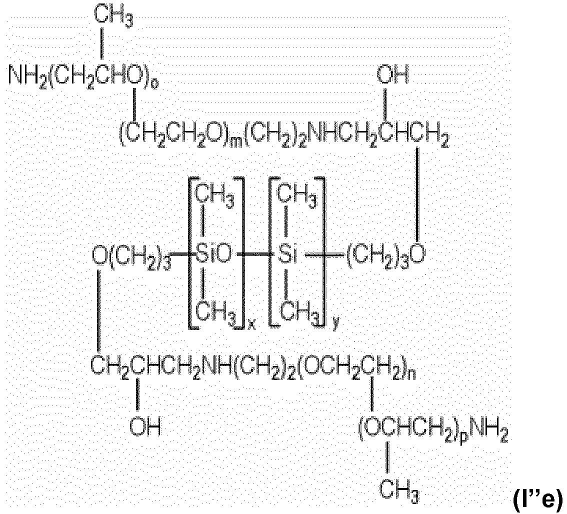 in which formula **(I"e)** m and n, which may be identical or different, represent an integer with the sum m + n having a mean value of 41 and o and p, which may be identical or different, represent an integer with the sum o + p having a mean value of 3.
   - that containing units of formula (lie) 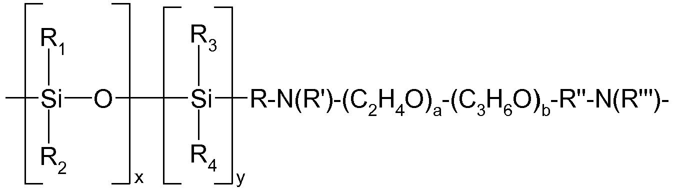 in which formula **(IIe)**
      - **R₁** to **R₄,** which may be identical or different, represent a C₁-C₄ alkyl radical, preferably methyl;
      - **R** and **R",** which may be identical or different, represent a linear or branched, optionally hydroxylated C₂-C₆ alkylene radical, optionally interrupted with an oxygen atom;
      - **a** and **b,** which may be identical or different, represent an integer ranging from 0 to 100;
      - **R'** and **R"',** which may be identical or different, represent a hydrogen atom or a C₁-C₄ alkyl radical such as a methyl radical; and
      - **x** denotes a number ranging from 1 to 500 and **y** denotes a number ranging from 1 to 10.

A compound falling within this category of amino silicones (e) is the product sold under the name Silsoft® A-843 by the company Momentive Performance Materials.

Among the amino silicones, mention may also be made of the silicone derived from the reaction between one or more copolymers of PEG-40/PPG-8 terminating with a 2-aminopropyl group and of bis-glycidoxypropyl dimethicone sold by the company Momentive Performance Materials under the name Silsoft® A+.

According to a particular embodiment of the invention, the amino silicones are used in combination with cationic and/or nonionic surfactants.

By way of example, use may be made of the products sold under the names Xiameter® MEM-939 Emulsion and Xiameter® MEM-949 Emulsion sold by the company Dow Corning, which comprise, besides amodimethicone, a cationic surfactant which is a trimethylcetylammonium halide (generally chloride) and a nonionic surfactant of formula C₁₃H₂₇-(OC₂H₄)₁₂-OH, known under the CTFA name Trideceth-12.

Another commercial product that may be used according to the invention is the product sold under the name Wacker-Belsil® ADM LOG 1, sold by the company Wacker, comprising, in microemulsion form, an amodimethicone of formula **(IIa)** in combination with Trideceth-5 and Trideceth-10.

Other amino silicones may be used in the context of the invention, such as the product referenced in the CTFA dictionary under the name Polysilicone-9.

Preferably, the amino silicone(s) used in the cosmetic composition according to the invention are chosen from amino silicones bearing polyalkoxylene groups consisting of polysiloxane block(s) and polyalkoxylene block(s) comprising at least one amine group, in particular those of formulae **(Ie), (I'e) and (I"e)** or those containing units **(IIe).**

The amino silicone(s) used in the composition according to the invention may be present in an amount ranging from 0.01% to 10%, better still from 0.1% to 5% by weight, preferably from 0.5% to 3% by weight, and even more preferentially from 0.5% to 1.5% by weight, relative to the total weight of the composition.

Preferably, the weight ratio between the amount of amino silicone(s), on the one hand, and the amount of dicarbonyl compounds corresponding to formula (I) and/or derivatives thereof, salts thereof and/or hydrates thereof ranges from 0.01 to 100, more preferentially from 0.01 to 20, better still from 0.05 to 10 and even better still from 0.05 to 1.

The compositions according to the invention can be provided in any galenical form conventionally used and in particular in the form of an aqueous, alcoholic or aqueous/alcoholic solution or suspension or oily solution or suspension; of a solution or a dispersion of the lotion or serum type; of an emulsion, in particular having a liquid or semi-liquid consistency, of the O/W, W/O or multiple type; of a suspension or emulsion having a soft consistency of (O/W) or (W/O) cream type; of an aqueous or anhydrous gel, or of any other cosmetic form.

These compositions can be packaged in pump-action sprays or in aerosol containers, in order to provide for application of the composition in the vaporized (lacquer) form or in the form of a mousse. Such packaging forms are indicated, for example, when it is desired to obtain a spray or a mousse, for the treatment of the hair. In these cases, the composition preferably comprises at least one propellant.

The compositions of the invention may be aqueous or anhydrous. They are preferably aqueous and then comprise water at a concentration ranging from 5% to 98%, better still from 5% to 90% and even better still from 10% to 90% by weight relative to the total weight of the composition.

The composition of the invention may also comprise at least one common cosmetic ingredient, chosen in particular from propellants; oils; solid fatty substances and in particular C₈-C₄₀ esters; C₈-C₄₀ acids; C₈-C₄₀ alcohols; surfactants; sunscreens; moisturizers; antidandruff agents; antioxidants; chelating agents; nacreous agents and opacifiers; plasticizers or coalescers; fillers; silicones other than the amino silicones and in particular polydimethylsiloxanes; polymeric or non-polymeric thickeners; gelling agents; emulsifiers; polymers, in particular conditioning or styling polymers; fragrances; basifying agents or acidifying agents; silanes; crosslinking agents. The composition can, of course, comprise several cosmetic ingredients appearing in the above list.

The composition may in particular comprise one or more organic solvents, in particular water-soluble solvents, such as C₁-C₇ alcohols; mention may in particular be made of C₁-C₇ aliphatic monoalcohols, C₆-C₇ aromatic monoalcohols, C₃-C₇ polyols or C₃-C₇ polyol ethers, which may be employed alone or as a mixture with water.

Depending on their nature and the purpose of the composition, the normal cosmetic ingredients can be present in normal amounts which can be easily determined by a person skilled in the art and which can be, for each ingredient, between 0.01% and 80% by weight. A person skilled in the art will take care to choose the ingredients included in the composition and the amounts thereof so that they do not harm the properties of the compositions of the present invention.

The pH of the composition is less than 4 and preferably ranges from 1 to 3, better still from 1.5 to 3 and even better still from 1.7 to 3.

It may be adjusted to the desired value by means of acidifying and/or basifying agents usually used for treating keratin fibres.

The basifying agent may be chosen from mineral or organic or hybrid alkaline agents, or mixtures thereof.

The mineral alkaline agent(s) are preferably chosen from aqueous ammonia, alkali metal carbonates or bicarbonates such as sodium or potassium carbonate and sodium or potassium bicarbonate, sodium hydroxide or potassium hydroxide, or mixtures thereof.

The organic alkaline agent(s) are preferably chosen from organic amines with a pKb at 25°C of less than 12, preferably less than 10 and even more advantageously less than 6. It should be noted that it is the pKb corresponding to the function of highest basicity.

Hybrid compounds that may be mentioned include the salts of the amines mentioned previously with acids such as carbonic acid or hydrochloric acid.
The organic alkaline agent(s) are chosen, for example, from amine derivatives such as alkanolamines, oxyethylenated and/or oxypropylenated ethylenediamines, amino acids of amines such as 1,3-diaminopropane, 1,3-diamino-2-propanol, spermine or spermidine.

The term "alkanolamine" means an organic amine comprising a primary, secondary or tertiary amine function, and one or more linear or branched C₁-C₈ alkyl groups bearing one or more hydroxyl radicals.

Sodium hydroxide is in particular suitable for use in the invention.

The acidifying agent may be chosen from mineral or organic acids, for instance hydrochloric acid, phosphoric acid or lactic acid.

The composition according to the invention is preferably in the form of styling or care gels, care lotions or creams, conditioners, masks or sera.

The composition according to the invention may be obtained by mixing several compositions.

The process of the invention comprises the application of the composition described previously, followed by a step of straightening the hair with an iron. Straightening with an iron is known from the prior art. It consists in straightening the hair with flat heating tongs, which are generally metallic. The straightening irons are generally used at a temperature ranging from 150 to 250°C.

The process of the invention may comprise other intermediate steps aimed at improving the straightening of the hair.

According to a particular embodiment, the process of the invention comprises the application of the composition of the invention to dry hair, and a time of contact of the composition with the hair of between 10 and 60 minutes, preferably between 20 and 40 minutes. After this leave-on time, straightening with a brush and with a hairdryer (blow-drying) is performed. The hair is then straightened with a straightening iron at a temperature of between 150 and 250°C, preferably from 210 to 230°C.

In another variant, the process for straightening keratin fibres, such as the hair, may comprise the successive application to the said fibres, and in any order with or without intermediate rinsing,
- of a composition comprising i) one or more dicarbonyl compounds corresponding to formula **(I)** and/or derivatives thereof and/or hydrates thereof and/or salts thereof as defined previously;
- of a composition comprising ii) one or more amino silicones as defined previously;
- being followed by a straightening step using a straightening iron at a temperature of at least 150°C, preferably ranging from 150 to 250°C.

According to one particular mode of the invention, i) is applied to the fibres, then ii).
According to another particular embodiment, ii) is applied first, and then i).

The process of the invention may also comprise the application of other hair agents as a pretreatment or post-treatment. In particular, it may comprise the application of a conditioning care product as a post-treatment.

According to another embodiment, the hair straightening process comprises a step of washing the hair and then of drying with a hairdryer before applying the composition of the invention. According to this particular embodiment, the steps described above are then encountered, such as the contact time of the composition, the blow-drying, the straightening with a straightening iron, the application of a conditioning agent and the rinsing, all these steps possibly being performed independently of each other. According to a particular embodiment, the straightening with the straightening iron is performed in several passes on the hair, in general 8 to 10 passes.

The process of the present invention is performed without a step of permanent reshaping at basic pH or based on a reducing agent.

### Examples

The composition below was prepared (weight % of starting material in unmodified form relative to the total weight of the composition):

| Ingredients | % |
|---|---|
| Hydroxypropylmethylcellulose (Methocel F4M from OW Chemical) | 1 |
| Glyoxylic acid (50% aqueous solution) | 16 |
| Disodium cocoamphodiacetate (sodium *N*-cocoylamidoethyl-*N-*ethoxycarbonylmethyl glycinate) as an aqueous 30% solution (Miranol C2M Conc. NP from Rhodia) | 2 |
| Polyquaternium-37(and) propylene glycol dicaprylate/dicaprate (and)PPG-1, Trideceth 6 (Salcare SC96 from BASF) | 2.5 |
| Lactic acid | 2.5 |
| Sodium hydroxide (10% aqueous solution) | 7 |
| Bisamino PEG/PPG-41-3 aminoethyl PG-propyl dimethicone at 30% AM in dipropylene glycol (Silsoft A 843 from Momentive Performance Materials) | 2 |
| Water | Qs |

| | |
|---|---|
| *pH 2.2 ± 0.2 | |

The above composition is applied to a lock of hair having a substantial level of curliness. After a leave-on time of 20 minutes on the hair, the lock is blow-dried with a hairdryer and is then straightened with a straightening iron. The evolution of fumes during the use of the iron is low.
The lock is then washed and dried with a hairdryer. A straight lock of hair free of curliness is thus obtained.

### Comparative example:

Compositions A and B were prepared with the following compositions :

| Compositions | A (invention) (%wt/wt) | B (Comparative) (%wt/wt) |
|---|---|---|
| GLYOXYLIC ACID (50% in water) | 5% AM | 5% AM |
| AMODIMETHICONE *(XIAMETER MEM-8299 EMULSION from Dow Corning)* | 0,86 % AM | - |
| SODIUM HYDROXIDE | QS pH 2.2 | QS pH 2.2 |
| DEIONIZED WATER | QS 100 | QS 100 |

2.7g hair locks of curl type IV bleached hair were washed with a shampoo and blow dried. Then 2.7g of composition A was applied to one of the hair lock and 2.7g of composition B was applied to another hair lock. After a leave-on time of 20 minutes on the hair, the locks were blow-dried with a hairdryer (brushing with 15 passes of a brush) and were then straightened with a straightening iron (10 passes). The locks were then washed with a shampoo and let air dried (spontaneous).

Qualities of use and conditioning performances were improved by using composition A.

In particular, the flat iron was easily applied on the hair lock treated with composition A. Hair was smoother and silkier after being treated by composition A. The hair lock treated with composition A was easier to comb than the hair lock treated with composition B on humid and dry hair.

Then, the hair locks were washed again with a second shampoo and then let air dried (spontaneous). Better lasting curl reduction was observed on the hair lock treated with composition A.

## Claims

1. Process for straightening keratin fibres such as the hair, which comprises the application to the said fibres of a cosmetic composition comprising:
i) one or more dicarbonyl compounds corresponding to formula (I) below, and/or derivatives thereof and/or hydrates thereof and/or salts thereof: 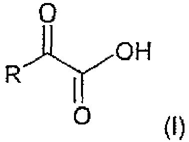 in which formula (I):
R represents an atom or group chosen from i) hydrogen, ii) carboxyl-C(O)OH, iii) linear or branched C₁-C₆ alkyl which is optionally substituted, preferably with at least one hydroxyl -OH radical, carboxyl -C(O)-OH radical or halogen such as Br, iv) optionally substituted phenyl, v) optionally substituted benzyl, iv) and v) preferably being optionally substituted with at least one -OH or -C(O)OH radical, vi) an indolyl radical and vii) an imidazolylmethyl radical and tautomers thereof such as: 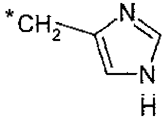 with * representing the part linked to the rest of the molecule,
"derivatives" of the dicarbonyl compounds of formula (I) being esters of the carboxyl group(s), amides of the carboxyl group(s), and (thio)acetals and hemi(thio)acetals of the carbonyl function(s) of the compounds of formula (I), in free form or optionally in the form of salts or hydrates, and
ii) at least one amino silicone,
the composition having a pH of less than or equal to 4,
followed by a straightening step using a straightening iron.

2. Process according to Claim 1, in which the dicarbonyl compound(s) are of formula (I) with **R** representing i) a hydrogen atom or ii) a linear or branched C₁-C₆ alkyl group optionally substituted with a carboxyl group.

3. Process according to either one of the preceding claims, in which the dicarbonyl compound(s) of formula (I) and/or derivatives thereof according to claim 1 and/or hydrates thereof and/or salts thereof are chosen from glyoxylic acid and pyruvic acid, a derivative thereof, salts thereof and hydrates thereof, preferably from glyoxylic acid, a derivative thereof according to claim 1 and the hydrate forms of these compounds.

4. Process according to any one of the preceding claims, in which the dicarbonyl compound(s) of formula **(I)** and/or derivatives thereof according to claim 1 are chosen from glyoxylic acid esters, glyoxylic acid amides, glyoxylic acid (thio)acetals and hemi(thio)acetals, and glyoxylic acid ester (thio)acetals and hemi(thio)acetals.

5. Process according to Claim 3, in which the glyoxylic acid is in its hydrate form.

6. Process according to any one of the preceding claims, in which the composition comprises from 0.1% to 20% by weight of one or more dicarbonyl compounds corresponding to formula **(I)** and/or derivatives thereof according to claim 1 and/or hydrates thereof and/or salts thereof, preferably from 3% to 10% by weight relative to the total weight of the composition.

7. Process according to any one of the preceding claims, in which the amino silicone(s) are chosen from the silicones ***(a)*** to ***(e)*** below:
***(a)*** the compounds corresponding to formula **(Ia)** below:
(R₁)ₐ(T)₃₋ₐ-Si[OSi(T)₂]ₙ-(OSi(T)_{b}(R₁)_{2-b}]ₘ-OSi(T)₃₋ₐ-(R₁)ₐ **(Ia)**
in which formula **(Ia):**
• **T** represents a hydrogen atom or a phenyl, hydroxyl (-OH) or C₁-Cₐ alkyl group, and preferably methyl, or a C₁-C₈ alkoxy, preferably methoxy;
• **a** denotes the number 0 or an integer from 1 to 3, and preferably 0;
• **b** denotes 0 or 1, and in particular 1;
• **m** and **n** are integers such that the sum (n + m) can range especially from 1 to 2000 and in particular from 50 to 150, it being possible for n to denote a number from 0 to 1999 and in particular from 49 to 149, and for m to denote a number from 1 to 2000 and in particular from 1 to 10;
• **R₁** is a monovalent group of formula -C_{q}H_{2q}L in which q is an integer from 2 to 8 and L is an optionally quaternized amino group chosen from the following groups:
-N(R₂)-CH₂-CH₂-N(R₂)₂,
-N(R₂)₂,
-N⁺(R₂)₃Q⁻,
-N⁺(R₂)(H)₂Q⁻,
-N⁺(R₂)₂HQ⁻,
-N(R₂)-CH₂-CH₂-N⁺(R₂)(H)₂Q⁻;
in which R₂ may denote a hydrogen atom, a phenyl group, a benzyl group or a saturated monovalent hydrocarbon-based group, for example a C₁-C₂₀ alkyl group, and Q⁻ represents an anionic counterion such as a halide ion, for instance fluoride, chloride, bromide or iodide;
***(b)*** the compounds corresponding to formula **(Ib)** below: 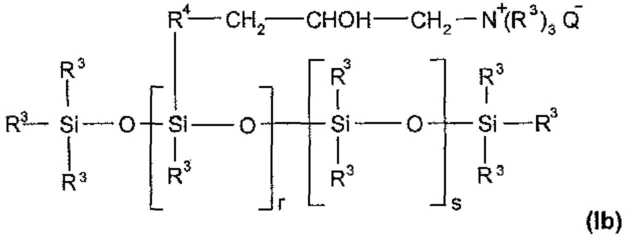 in which formula **(Ib):**
• **R³** represents a monovalent C₁-C₁₈ hydrocarbon-based group, and in particular a C₁-C₁₈ alkyl or C₂-C₁₈ alkenyl group, for example methyl;
• **R⁴** represents a divalent hydrocarbon-based group, in particular a C₁-C₁₈ alkylene group or a divalent C₁-C₁₈, for example C₁-C₈, alkylenoxy group;
• **Q⁻** represents an anionic counterion such as that chosen from halide ions, especially chloride;
• **r** represents a mean statistical value from 2 to 20 and in particular from 2 to 8;
• **s** represents a mean statistical value between 20 and 200 inclusive and in particular between 20 and 50 inclusive;
***(c)*** the quaternary ammonium silicones, especially of formula **(Ic):** 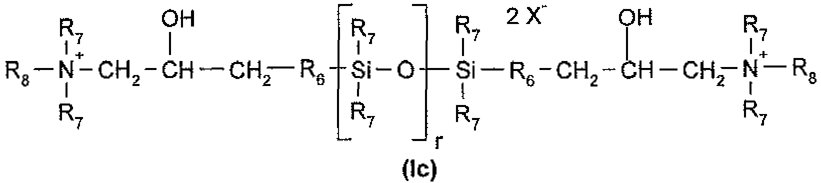 in which formula **(Ic):**
• **R₆** represents a divalent hydrocarbon-based group, especially a C₁-C₁₈ alkylene group or a divalent C₁-C₁₈, for example C₁-C₈, alkylenoxy group linked to the Si atom via an Si-C bond;
• **R₇,** which may be identical or different, represent a monovalent hydrocarbon-based group containing from 1 to 18 carbon atoms, and in particular a C₁-C₁₈ alkyl group, a C₂-C₁₈ alkenyl group or a ring comprising 5 or 6 carbon atoms, for example methyl;
• **R₈,** which may be identical or different, each represent a hydrogen atom, a monovalent hydrocarbon-based group containing from 1 to 18 carbon atoms, and in particular a C₁-C₁₈ alkyl group, a C₂-C₁₈ alkenyl group or a group -R₆-N(H)-C(O)-R₇ with R₆ and R₇ as defined previously;
• **X⁻,** which may be identical or different, represents an anionic counterion such as a halide ion, especially chloride, or an anionic counterion derived from an organic acid such as (C₁-C₆)alkylcarboxylate;
• **r** represents a mean statistical value between 2 and 200 inclusive and in particular between 5 and 100;
***(d)*** the amino silicones of formula **(Id):** 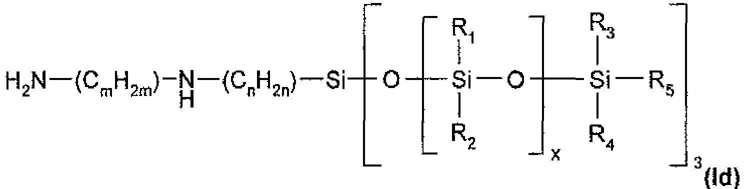 in which formula **(Id):**
• **R₁, R₂, R₃** and **R₄,** which may be identical or different, each denote a C₁-C₄ alkyl group or an aryl group such as phenyl,
• **R₅** denotes a C₁-C₄ alkyl group or a hydroxyl group,
• **n and m,** which may be identical or different, represent an integer between 1 and 5 inclusive, and
• **x** is such that the amine number is between 0.01 and 1 meq/g;
***(e)*** the amino silicones containing polyalkoxylene groups of formula **(Ie):** 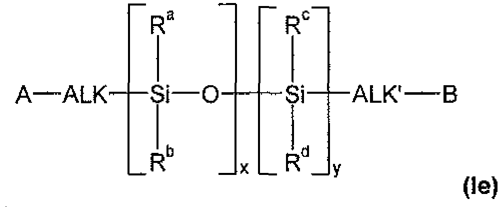 in which formula **(Ie):**
• **R^{a}, R^{b}, R^{c}** and **R^{d},** which may be identical or different, represent a hydroxyl or linear or branched (C₁-C₁₀)alkyl group, and preferably **R^{a}, R^{b}, R^{c}** and **R^{d}** represent a (C₁-C₆)alkyl group, which is more particularly linear, such as methyl;
• **ALK** and **ALK',** which may be identical or different, represent a linear or branched (C₁-C₁₀)alkylene group, which is preferably linear, such as propylene;
• **A** and **B,** which may be identical or different, represent an aminopolyalkoxy group below:
R^{e}R^{f}N-[ALK"-O]_{z}-[ALK"-O]_{w}-ALKₐ-N(R^{g})-[ALK_{b}-O]_{q}-*
with:
- * representing the point of attachment of the radical to the rest of the molecule via ALK or ALK';
- **R^{e}, R^{f}** and **R^{g},** which may be identical or different, representing a hydrogen atom or a linear or branched (C₁-C₁₀)alkyl group, and preferably **R^{e}, R^{f}** and **R^{g}** represent a hydrogen atom;
- **ALK"** and **ALK"',** which may be identical or different, represent a linear or branched (C₁-C₁₀)alkylene group, preferably of C₂ or C₃; more particularly, ALK" represents a divalent group -CH₂-CH(CH₃)- and ALK'" represents an ethylene group;
- **ALKₐ** and **ALK_{b},** which may be identical or different, represent a linear or branched (C₁-C₁₀)alkylene group, which is optionally substituted preferably with a hydroxyl group, and is preferably of C₂ or C₃; more particularly, ALKₐ represents an ethylene or propylene group or a divalent group -CH₂-CH(CH₃)- and ALK_{b} represents a divalent group -CH₂-CH(OH)-CH₂-;
- q, which may be identical or different, represent 0 or 1, preferably 1;
- w, which may be identical or different, represent an integer, preferably the sum of the w values (w of A + w of B) having a mean value inclusively between 10 and 100, more particularly inclusively between 20 and 60 and more preferentially between 30 and 50, such as 40-41;
- z, which may be identical or different, represent an integer, preferably the sum of the z values (z of A + z of B) having a mean value inclusively between 1 and 20, more particularly inclusively between 1 and 10 and more preferentially between 2 and 5, such as 3;
***(I'e)*** the amino silicones containing polyalkoxylene groups consisting of polysiloxane block(s) and of polyalkoxylene block(s) comprising at least one amine group, in particular:
- those of formula **(I'e):** 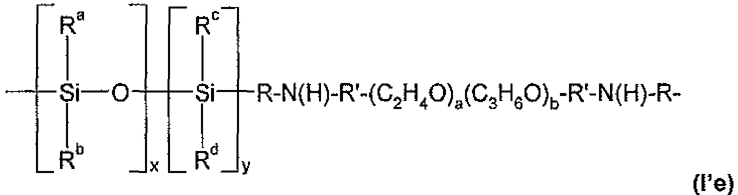 in which formula **(I'e):**
• **R^{a}, R^{b}, R^{c}** and **R^{d},** which may be identical or different, represent a hydroxyl or linear or branched (C₁-C₁₀)alkyl group, and preferably **R^{a}, R^{b}, R^{c}** and **R^{d}** represent a (C₁-C₄)alkyl group, which is more particularly linear, such as methyl;
• **R** and **R",** which may be identical or different, represent a linear or branched, optionally hydroxylated C₂-C₆ alkylene radical, optionally interrupted with an oxygen atom;
• **a** and **b,** which may be identical or different, represent a number ranging from 0 to 100;
• **R'** and **R"',** which may be identical or different, represent a hydrogen atom or a C₁-C₄ alkyl radical such as a methyl radical;
• **x** denotes an integer ranging from 1 to 500 and **y** denotes an integer ranging from 1 to 10;
- that containing units of formula **(IIe)** 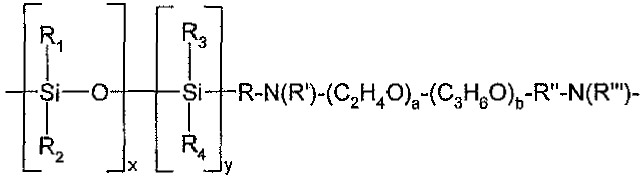 in which formula **(IIe)**
• **R₁** to **R₄,** which may be identical or different, represent a C₁-C₄ alkyl radical, preferably methyl;
• **R** and **R",** which may be identical or different, represent a linear or branched, optionally hydroxylated C₂-C₆ alkylene radical, optionally interrupted with an oxygen atom;
• **a** and **b,** which may be identical or different, represent an integer ranging from 0 to 100;
• **R'** and **R"',** which may be identical or different, represent a hydrogen atom or a C₁-C₄ alkyl radical such as a methyl radical; and
• **x** denotes a number ranging from 1 to 500 and **y** denotes a number ranging from 1 to 10.

8. Process according to any one of the preceding claims, in which the amino silicone(s) are chosen from amino silicones bearing polyalkoxylene groups consisting of polysiloxane block(s) and polyalkoxylene block(s) comprising at least one amine group, in particular those of formula **(Ie)** or **(Ie')** as defined in the preceding claim, and particularly the amino silicone(s) are chosen from:
i) the amino silicones bisamino PolyEthyleneGlycol/PolyPropyleneGlycol-41-3 aminoethyl PG(glycidopropyl)-propyl dimethicone in particular of formula **(IIe)** below: 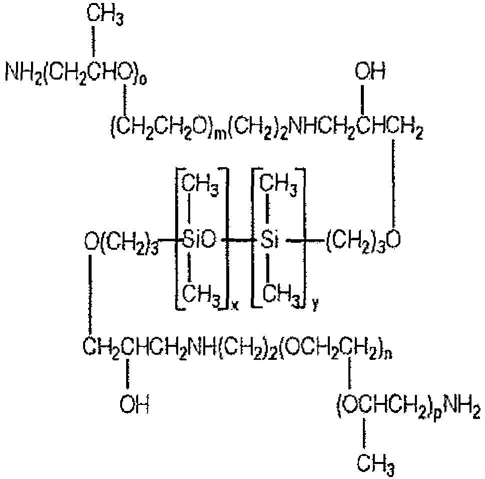 in which formula **(lie)** m and n, which may be identical or different, represent an integer with the sum m + n having a mean value of 41 and o and p, which may be identical or different, represent an integer with the sum o + p having a mean value of 3.

9. Process according to any one of the preceding claims, in which the amino sificone(s) are chosen from amino silicones derived from the reaction between one or more copolymers of PEG-40/PPG-8 terminating with a 2-aminopropyl group and of bis-glycidoxypropyl dimethicone.

10. Process according to any one of the preceding claims, in which the amino silicone(s) are present in an amount ranging from 0.01% to 10%, better still from 0.1% to 5% by weight, preferably from 0.5% to 3% by weight and even more preferentially from 0.5% to 1.5% by weight relative to the total weight of the composition.

11. Process according to any one of the preceding claims, in which the weight ratio between the amount of amino silicone(s) as defined in Claims 1 and 7 to 9, on the one hand, and the amount of dicarbonyl compounds corresponding to formula (I) and/or derivatives thereof according to claim 1 and/or hydrates thereof and/or salts thereof as defined in Claims 1 to 6 ranges from 0.01 to 100, even more preferentially from 0.01 to 20, better still from 0.05 to 10 and even better still from 0.05 to 1.

12. Process according to any one of the preceding claims, **characterized in that** it is aqueous and comprises water in a concentration preferably ranging from 5% to 98%, better still from 5% to 90% and even better still from 10% to 90% by weight relative to the total weight of the composition.

13. Process according to any one of the preceding claims, **characterized in that** the composition results from the mixing of several compositions.

14. Process according to any one of the preceding claims, wherein the time of contact of the composition with the hair is of between 10 and 60 minutes.

15. Process according to any one of the preceding claims, wherein the hair is straightened using a straightening iron at a temperature of at least 150°C and preferably between 150 and 250°C.

16. Use of a composition comprising:
i) one or more dicarbonyl compounds corresponding to formula (I) below, and/or derivatives thereof and/or hydrates thereof and/or salts thereof: 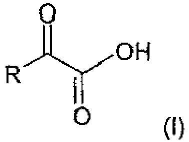 in which formula **(I):**
**R** represents an atom or group chosen from i) hydrogen, ii) carboxyl-C(O)OH, iii) linear or branched C₁-C₆ alkyl which is optionally substituted, preferably with at least one hydroxyl -OH radical, carboxyl -C(O)-OH radical or halogen such as Br, iv) optionally substituted phenyl, v) optionally substituted benzyl, iv) and v) preferably being optionally substituted with at least one -OH or -C(O)OH radical, vi) an indolyl radical and vii) an imidazolylmethyl radical and tautomers thereof such as: 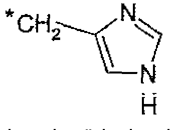 with * representing the part linked to the rest of the molecule, derivatives of the dicarbonyl compounds of formula (I) being esters of the carboxyl group(s), amides of the carboxyl group(s), and (thio)acetals and hemi(thio)acetals of the carbonyl function(s) of the compounds of formula (I), in free form or optionally in the form of salts or hydrates, and
ii) at least one amino silicone,
the composition having a pH of less than or equal to 4, for straightening/relaxing keratin fibres, especially the hair.

17. Cosmetic composition, in particular a hair composition, comprising
i) one or more dicarbonyl compounds corresponding to formula (I) below, and/or derivatives thereof and/or hydrates thereof and/or salts thereof: 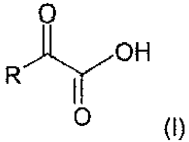 in which formula **(I):**
R represents an atom or group chosen from i) hydrogen, ii) carboxyl -C(O)OH, iii) linear or branched C₁-C₆ alkyl or linear or branched C₁-C₆ alkyl substituted with at least one hydroxyl -OH radical or linear or branched C₁-C₆ alkyl substituted with halogen such as Br, iv) optionally substituted phenyl, v) optionally substituted benzyl, iv) and v) preferably being optionally substituted with at least one -OH or - C(O)OH radical, vi) an indolyl radical and vii) an imidazolylmethyl radical and tautomers thereof such as 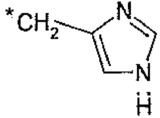 with * representing the part linked to the rest of the molecule, "derivatives" of the dicarbonyl compounds of formula (I) being esters of the carboxyl group(s), amides of the carboxyl group(s), and (thio)acetals and hemi(thio)acetals of the carbonyl function(s) of the compounds of formula (I), in free form or optionally in the form of salts or hydrates, preferably one or more dicarbonyl compounds according to any one of claims 3 to 6,
and ii) at least one amino silicone, preferably at least one amino silicone according to any one of claims 7 to 10, the composition having a pH of less than or equal to 4.

## Patentansprüche

1. Verfahren zum Glätten von Keratinfasern, insbesondere des Haars, umfassend das Auftragen einer kosmetischen Zusammensetzung, umfassend:
i) eine oder mehrere Dicarbonylverbindungen gemäß nachstehender Formel (I) und/oder Derivate davon und/oder Hydrate davon und/oder Salze davon: 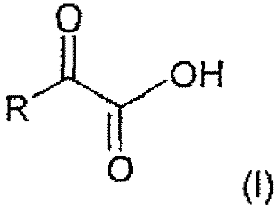 wobei in Formel (I):
**R** für ein Atom oder eine Gruppe steht, das bzw. die ausgewählt ist aus: i) Wasserstoff, ii) Carboxyl -C(O)OH, iii) geradkettigem oder verzweigtem C₁-C₆-Alkyl, das gegebenenfalls substituiert ist, vorzugsweise durch mindestens einen Hydroxylrest -OH, einen Carboxylrest -C(O)OH oder ein Halogen wie Br; iv) gegebenenfalls substituiertem Phenyl, v) gegebenenfalls substituiertem Benzyl, wobei iv) und v) vorzugsweise gegebenenfalls durch mindestens einen -OH- oder -C(O)OH-Rest substituiert sind; vi) einem Indolylrest und vii) einem Imidazolylmethylrest und Tautomeren davon wie 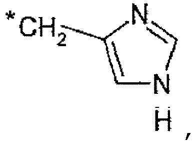 wobei * für den mit dem Rest des Moleküls verknüpften Teil steht, wobei es sich bei "Derivaten" der Dicarbonylverbindungen der Formel (I) um Ester der Carboxylgruppe(n), Amide der Carboxylgruppe(n) und (Thio)acetale und Hemi(thio)acetale der Carbonylfunktion(en) der Verbindungen der Formel (I) in freier Form oder gegebenenfalls in Form von Salzen oder Hydraten handelt, und
ii) mindestens ein Aminosilikon,
wobei die Zusammensetzung einen pH-Wert kleiner oder gleich 4 aufweist,
auf die Fasern und anschließend einen Glättungsschritt unter Verwendung eines Glätteisens.

2. Verfahren nach Anspruch 1, wobei die Dicarbonylverbindung(en) die Formel **(I)** aufweist bzw. aufweisen, wobei **R** für i) ein Wasserstoffatom oder ii) eine geradkettige oder verzweigte C₁-C₆-Alkylgruppe, die gegebenenfalls durch eine Carboxylgruppe substituiert ist, steht.

3. Verfahren nach einem der vorhergehenden Ansprüche, wobei die Dicarbonylverbindung(en) der Formel **(I)** und/oder Derivate davon nach Anspruch 1 und/oder Hydrate davon und/oder Salze davon aus Glyoxylsäure und Brenztraubensäure, einem Derivat davon, Salzen davon und Hydraten davon, vorzugsweise aus Glyoxylsäure, einem Derivat davon nach Anspruch 1 und den Hydratformen dieser Verbindungen, ausgewählt ist bzw. sind.

4. Verfahren nach einem der vorhergehenden Ansprüche, wobei die Dicarbonylverbindung(en) der Formel **(I)** und/oder Derivate davon nach Anspruch 1 aus Glyoxylsäureestern, Glyoxylsäureamiden, Glyoxylsäure(thio)acetalen und -hemi(thio)acetalen und Glyoxylsäureester(thio)acetalen und -hemi(thio)-acetalen ausgewählt ist bzw. sind.

5. Verfahren nach Anspruch 3, wobei die Glyoxylsäure in ihrer Hydratform vorliegt.

6. Verfahren nach einem der vorhergehenden Ansprüche, wobei die Zusammensetzung 0,1 bis 20 Gew.-% einer oder mehrere Dicarbonylverbindungen der Formel **(I)** und/oder Derivate davon nach Anspruch 1 und/oder Hydrate davon und/oder Salze davon, vorzugsweise 3 bis 10 Gew.-%, bezogen auf das Gesamtgewicht der Zusammensetzung, umfasst.

7. Verfahren nach einem der vorhergehenden Ansprüche, wobei das Aminosilikon bzw. die Aminosilikone aus den nachstehenden Silikonen ***(a)*** bis ***(e)*** ausgewählt ist bzw. sind:
***(a)*** den Verbindungen der folgenden Formel **(Ia):**
(R₁)ₐ(T)₃₋ₐ-Si[OSi(T)₂]ₙ-[OSi(T)_{b}(R₁)_{2-b}]ₘ-OSi(T)₃₋ₐ-(R₁)ₐ **(Ia)**
wobei in der Formel **(Ia):**
• **T** für ein Wasserstoffatom oder eine Phenyl-, Hydroxyl(-OH)- oder C₁-C₈-Alkylgruppe und vorzugsweise für Methyl oder eine C₁-C₈-Alkoxy, vorzugsweise Methyl, steht,
• **a** für die Zahl 0 oder eine ganze Zahl von 1 bis 3 und vorzugsweise 0 steht,
• **b** für 0 oder 1 und insbesondere 1 steht,
• **m** und **n** für solche ganzen Zahlen stehen, dass die Summe (n + m) insbesondere von 1 bis 2000 und speziell von 50 bis 150 variieren kann, wobei n eine Zahl von 0 bis 1999 und insbesondere von 49 bis 149 bedeuten kann und m eine Zahl von 1 bis 2000 und insbesondere 1 bis 10 bedeuten kann;
• **R₁** für einen einwertigen Rest der Formel -C_{q}H_{2q}L steht, in der q eine Zahl von 2 bis 8 bedeutet und L eine gegebenenfalls quaternisierte Aminogruppe bedeutet, die aus den folgenden Gruppen ausgewählt ist:
-N(R₂)-CH₂-CH₂-N(R₂)₂;
-N(R₂)2;
-N⁺(R₂)₃Q⁻;
-N⁺(R₂)(H)₂Q⁻;
-N⁺(R₂)₂HQ⁻;
-N(R₂)-CH₂-CH₂-N⁺(R₂)(H)₂Q⁻;
in denen R₂ für ein Wasserstoffatom, eine Phenylgruppe, eine Benzylgruppe oder eine einwertige gesättigte Gruppe auf Kohlenwasserstoffbasis, beispielsweise eine C₁-C₂₀-Alkylgruppe, stehen kann und Q⁻ für ein anionisches Gegenion wie ein Halogenidion, beispielsweise Fluorid, Chlorid, Bromid oder Iodid, steht;
***(b)*** den Verbindungen der nachstehenden Formel **(Ib)** : 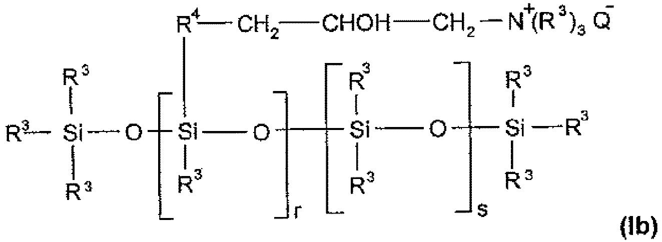 wobei in der Formel **(Ib):**
• **R³** für eine einwertige C₁-C₁₈-Gruppe auf Kohlenwasserstoffbasis und insbesondere eine C₁-C₁₈-Alkylgruppe oder C₂-C₁₈-Alkenylgruppe, beispielsweise Methyl, steht;
• **R⁴** für eine zweiwertige Gruppe auf Kohlenwasserstoffbasis, insbesondere eine C₁-C₁₈-Alkylengruppe oder eine zweiwertige C₁-C₁₈-Alkylenoxygruppe, beispielsweise C₁-C₈-Alkylenoxygruppe, steht;
• **Q⁻** für ein anionisches Gegenion wie dasjenige, das aus Halogenidionen ausgewählt ist, insbesondere Chlorid, steht;
• **r** für einen mittleren statistischen Wert von 2 bis 20 und insbesondere von 2 bis 8 steht;
• **s** für einen mittleren statistischen Wert zwischen 20 und 200 inklusive und insbesondere zwischen 20 und 50 inklusive steht;
***(c)*** den quaternären Ammoniumsilikonen, insbesondere der Formel **(Ic):** 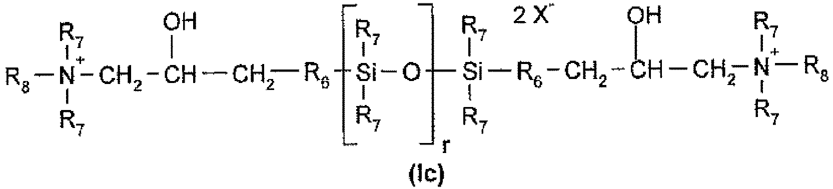 wobei in der Formel **(Ic):**
• **R₆** für eine zweiwertige Gruppe auf Kohlenwasserstoffbasis, insbesondere eine C₁-C₁₈-Alkylengruppe oder eine zweiwertige C₁-C₁₈-Alkylenoxygruppe, beispielsweise C₁-C₈-Alkylenoxygruppe, die über eine Si-C-Bindung an das Si-Atom gebunden ist, steht;
• die Reste **R₇** gleich oder verschieden sein können und für eine einwertige Gruppe auf Kohlenwasserstoffbasis mit 1 bis 18 Kohlenstoffatomen und insbesondere eine C₁-C₁₈-Alkylgruppe, eine C₂-C₁₈-Alkenylgruppe oder einen Ring mit 5 oder 6 Kohlenstoffatomen, beispielsweise Methyl, stehen;
• die Reste **R₈,** die gleich oder verschieden sein können, jeweils für ein Wasserstoffatom, eine einwertige Gruppe auf Kohlenwasserstoffbasis mit 1 bis 18 Kohlenstoffatomen und insbesondere eine C₁-C₁₈-Alkylgruppe, eine C₂-C₁₈-Alkenylgruppe oder eine Gruppe -R₆-N(H)-C(O)-R₇, wobei R₆ und R₇ wie oben definiert sind, stehen;
• die Variablen **X**⁻**,** die gleich oder verschieden sein können, für ein anionisches Gegenion wie ein Halogenidion, insbesondere Chlorid, oder ein anionisches Gegenion, das sich von einer organischen Säure ableitet, wie (C₁-C₆)Alkylcarboxylat steht;
• **r** für einen mittleren statistischen Wert von 2 bis 200 und insbesondere von 5 bis 100 steht;
***(d)*** den Aminosilikonen der Formel **(Id):** 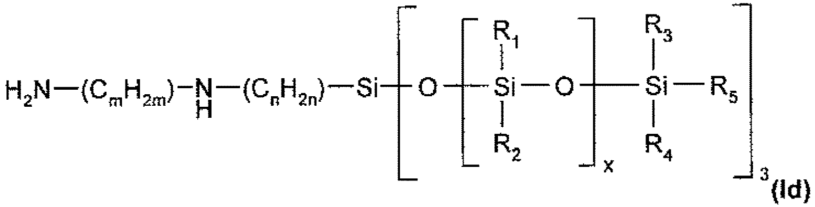 wobei in der Formel **(Id):**
• **R₁, R₂, R₃** und **R₄,** die gleich oder verschieden sein können, für eine C₁-C₄-Alkylgruppe oder eine Arylgruppe wie Phenyl stehen,
• **R₅** für eine C₁-C₄-Alkylgruppe oder eine Hydroxylgruppe steht,
• **n** und **m,** die gleich oder verschieden sein können, für eine ganze Zahl im Bereich zwischen 1 und 5 inklusive stehen und
• **x** so gewählt ist, dass die Aminzahl zwischen 0,01 und 1 meq/g liegt;
***(e)*** den Aminosilikonen mit Polyalkoxylengruppen der Formel (Ie): 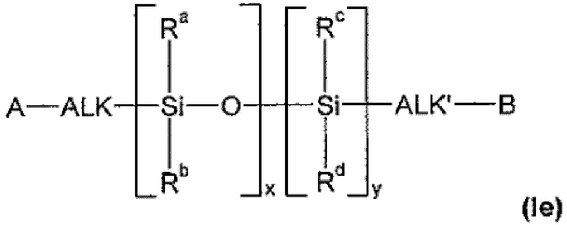 wobei in der Formel **(Ie):**
• **R^{a}, R^{b}, R^{c}** und **R^{d},** die gleich oder verschieden sein können,für ein Hydroxyl oder eine lineare oder verzweigte (C₁-C₁₀) Alkylgruppe stehen und vorzugsweise **R^{a}, R^{b}, R^{c}** und **R^{d}** für eine (C₁-C₆)-Alkylgruppe, die spezieller linear ist, wie Methyl, stehen;
• **ALK** und **ALK',** die gleich oder verschieden sein können, für eine lineare oder verzweigte (C₁-C₁₀)Alkylengruppe, die vorzugsweise linear ist, wie Propylen, stehen;
• **A** und **B,** die gleich oder verschieden sein können, für eine nachstehende Aminopolyalkoxygruppe steht:
R^{e}R^{f}N-[ALK"-O]_{z}-[ALK"'-O]_{w}-ALKₐ-N(R^{g})-[ALK_{b}-O]_{q}-*
wobei:
- * für den Punkt der Verknüpfung des Rests mit dem Rest des Moleküls über ALK bzw. ALK' steht;
- **R^{e}, R^{f}** und **R^{g},** die gleich oder verschieden sein können, für ein Wasserstoffatom oder eine lineare oder verzweigte (C₁-C₁₀)-Alkylgruppe stehen und vorzugsweise **R^{e}, R^{f}** und **R^{g}** für ein Wasserstoffatom stehen;
- **ALK"** und **ALK"',** die gleich oder verschieden sein können, für eine lineare oder verzweigte (C₁-C₁₀)Alkylengruppe, vorzugsweise eine C₂- oder C₃-Alkylengruppe, stehen; weiter bevorzugt ALK" für eine zweiwertige Gruppe -CH₂-CH(CH₃)- steht und ALK"' für eine Ethylengruppe steht;
- **ALKₐ** und **ALK_{b},** die gleich oder verschieden sein können, für eine lineare oder verzweigte (C₁-C₁₀)Alkylengruppe, die gegebenenfalls substituiert ist, vorzugsweise durch eine Hydroxylgruppe, und vorzugsweise eine C₂- oder C₃-Alkylengruppe, stehen; weiter bevorzugt ALKₐ für eine zweiwertige Gruppe -CH₂-CH(CH₃)-steht und ALK_{b} für eine zweiwertige Gruppe - CH₂-CH(OH)-CH₂- steht;
- die Variablen **q,** die gleich oder verschieden sein können, für 0 oder 1, vorzugsweise 1, stehen;
- die Variablen **w,** die gleich oder verschieden sein können, für eine ganze Zahl stehen, wobei die Summe der w-Werte (w von A + w von B) vorzugsweise einen mittleren Wert zwischen 10 und 100 inklusive, spezieller zwischen 20 und 60 inklusive und weiter bevorzugt zwischen 30 und 50, wie 40-41, aufweist;
- die Variablen **z,** die gleich oder verschieden sein können, für eine ganze Zahl stehen, wobei die Summe der z-Werte (z von A + z von B) vorzugsweise einen mittleren Wert zwischen 1 und 20 inklusive, spezieller zwischen 1 und 10 inklusive und weiter bevorzugt zwischen 2 und 5, wie 3, aufweist;
***(I'e)*** den Aminosilikonen mit Polyalkoxylengruppen aus einem oder mehreren Polysiloxanblöcken und einem oder mehreren Polyalkoxylenblöcken mit mindestens einer Amingruppe, insbesondere:
- denjenigen der Formel **(I'e):** 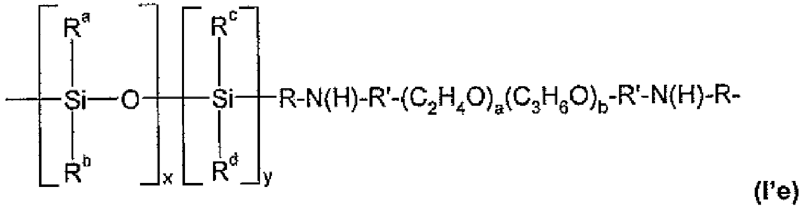 wobei in der Formel **(I'e):**
• **R^{a}, R^{b}, R^{c}** und **R^{d},** die gleich oder verschieden sein können, für ein Hydroxyl oder eine lineare oder verzweigte (C₁-C₁₀) Alkylgruppe stehen und vorzugsweise **R^{a}, R^{b}, R^{c}** und **R^{d}** für eine (C₁-C₄)-Alkylgruppe, die spezieller linear ist, wie Methyl, stehen;
• **R** und **R",** die gleich oder verschieden sein können, für einen linearen oder verzweigten, gegebenenfalls hydroxylierten C₂-C₆-Alkylenrest, der gegebenenfalls durch ein Sauerstoffatom unterbrochen ist, stehen;
• **a** und **b,** die gleich oder verschieden sein können, für eine Zahl im Bereich von 0 bis 100 stehen;
• **R'** und **R"',** die gleich oder verschieden sein können, für ein Wasserstoffatom oder einen C₁-C₄-Alkylrest wie einen Methylrest stehen;
• **x** für eine ganze Zahl im Bereich von 1 bis 500 steht **y** für eine ganze Zahl im Bereich von 1 bis 10 steht;
- demjenigen mit Einheiten der Formel **(IIe)** 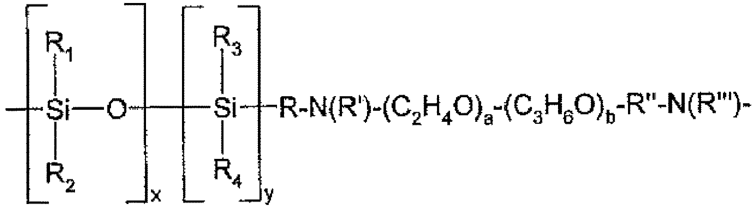 wobei in der Formel **(IIe):**
• **R¹** bis **R⁴,** die gleich oder verschieden sein können, für einen C₁-C₄-Alkylrest, vorzugsweise Methyl, stehen;
• **R** und **R",** die gleich oder verschieden sein können, für einen linearen oder verzweigten, gegebenenfalls hydroxylierten C₂-C₆-Alkylenrest, der gegebenenfalls durch ein Sauerstoffatom unterbrochen ist, stehen;
• **a** und **b,** die gleich oder verschieden sein können, für eine Zahl im Bereich von 0 bis 100 stehen;
• **R'** und **R"',** die gleich oder verschieden sein können, für ein Wasserstoffatom oder einen C₁-C₄-Alkylrest wie einen Methylrest stehen; und
• **x** für eine ganze Zahl im Bereich von 1 bis 500 steht und **y** für eine ganze Zahl im Bereich von 1 bis 10 steht.

8. Verfahren nach einem der vorhergehenden Ansprüche, wobei das Aminosilikon bzw. die Aminosilikone aus Silikonen mit Polyalkoxylengruppen aus einem oder mehreren Polysiloxanblöcken und einem oder mehreren Polyalkoxylenblöcken mit mindestens einer Amingruppe, insbesondere denjenigen der Formel **(Ie)** oder **(Ie')** gemäß dem vorhergehenden Anspruch, ausgewählt ist bzw. sind und das Aminosilikon bzw. die Aminoslikone aus:
i) den Aminosilikonen BisaminoPolyEthylen-Glykol/PolyPropylenGlykol-41-3-aminoethyl-PG-(glycidopropyl)propyldimethicon, insbesondere der nachstehenden Formel **(IIe):** 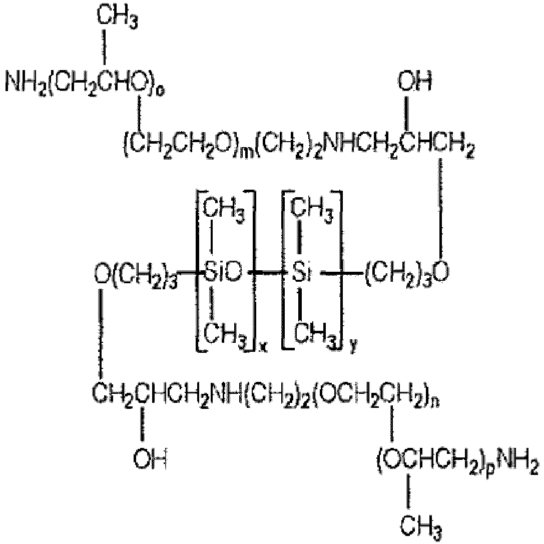 wobei in Formel **(IIe)** m und n, die gleich oder verschieden sein können, für eine ganze Zahl stehen, wobei die Summe m + n einen mittleren Wert von 41 aufweist, und o und p, die gleich oder verschieden sein können, für eine ganze Zahl stehen, wobei die Summe o + p einen mittleren Wert von 3 aufweist,
ausgewählt ist bzw. sind.

9. Verfahren nach einem der vorhergehenden Ansprüche, wobei das Aminosilikon bzw. die Aminosilikone aus Aminosilikonen aus der Umsetzung zwischen einem oder mehreren Copolymeren von PEG-40/PPG-8 mit einer 2-aminopropylendgruppe und Bis-glycidoxypropyldimethicon ausgewählt ist bzw. sind.

10. Verfahren nach einem der vorhergehenden Ansprüche, wobei das Aminosilikon bzw. die Aminosilikone in einer Menge im Bereich von 0,01 bis 10 Gew.-%, noch besser von 0,1 bis 5 Gew.-%, vorzugsweise von 0,5 bis 3 Gew.-% und noch weiter bevorzugt von 0,5 bis 1,5 Gew.-%, bezogen auf das Gesamtgewicht der Zusammensetzung, vorliegt bzw. vorliegen.

11. Verfahren nach einem der vorhergehenden Ansprüche, wobei das Gewichtsverhältnis zwischen der Menge von Aminosilikon(en) gemäß den Ansprüchen 1 und 7 bis 9 einerseits und der Menge von Dicarbonylverbindungen der Formel (I) und/oder Derivaten davon nach Anspruch 1 und/oder Hydraten davon und/oder Salzen davon gemäß den Ansprüchen 1 bis 6 im Bereich von 0,01 bis 100, noch weiter bevorzugt von 0,01 bis 20, noch besser von 0,05 bis 10 und noch besser von 0,05 bis 1 liegt.

12. Verfahren nach einem der vorhergehenden Ansprüche, **dadurch gekennzeichnet, dass** es wässrig ist und Wasser in einer Konzentration, die vorzugsweise im Bereich von 5 bis 98 Gew.-%, noch besser von 5 bis 90 Gew.-%, noch besser von 10 bis 90 Gew.-%, bezogen auf das Gesamtgewicht der Zusammensetzung, liegt, umfasst.

13. Verfahren nach einem der vorhergehenden Ansprüche, **dadurch gekennzeichnet, dass** sich die Zusammensetzung aus dem Mischen mehrerer Zusammensetzungen ergibt.

14. Verfahren nach einem der vorhergehenden Ansprüche, wobei die Kontaktzeit der Zusammensetzung mit dem Haar zwischen 10 und 60 Minuten liegt.

15. Verfahren nach einem der vorhergehenden Ansprüche, wobei das Haar mit einem Glätteisen bei einer Temperatur von mindestens 150°C und vorzugsweise zwischen 150 und 250 °C geglättet wird.

16. Verwendung einer Zusammensetzung, umfassend:
i) eine oder mehrere Dicarbonylverbindungen gemäß nachstehender Formel (I) und/oder Derivate davon und/oder Hydrate davon und/oder Salze davon: 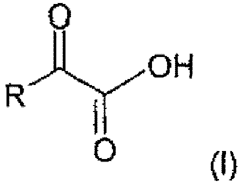 wobei in Formel (I):
R für ein Atom oder eine Gruppe steht, das bzw. die ausgewählt ist aus: i) Wasserstoff, ii) Carboxyl -C(O)OH, iii) geradkettigem oder verzweigtem C₁-C₆-Alkyl, das gegebenenfalls substituiert ist, vorzugsweise durch mindestens einen Hydroxylrest -OH, einen Carboxylrest -C(O)OH oder ein Halogen wie Br; iv) gegebenenfalls substituiertem Phenyl, v) gegebenenfalls substituiertem Benzyl, wobei iv) und v) vorzugsweise gegebenenfalls durch mindestens einen -OH- oder -C(O)OH-Rest substituiert sind; vi) einem Indolylrest und vii) einem Imidazolylmethylrest und Tautomeren davon wie 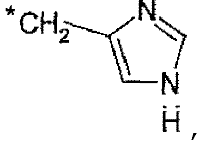 wobei * für den mit dem Rest des Moleküls verknüpften Teil steht, wobei es sich bei "Derivaten" der Dicarbonylverbindungen der Formel (I) um Ester der Carboxylgruppe(n), Amide der Carboxylgruppe(n) und (Thio)acetale und Hemi(thio)acetale der Carbonylfunktion(en) der Verbindungen der Formel (I) in freier Form oder gegebenenfalls in Form von Salzen oder Hydraten handelt,
und
ii) mindestens ein Aminosilikon,
wobei die Zusammensetzung einen pH-Wert kleiner oder gleich 4 aufweist, zum Glätten/Entkräuseln von Keratinfasern, insbesondere dem Haar.

17. Kosmetische Zusammensetzung, insbesondere Haarzusammensetzung, umfassend:
i) eine oder mehrere Dicarbonylverbindungen gemäß nachstehender Formel (I) und/oder Derivate davon und/oder Hydrate davon und/oder Salze davon: 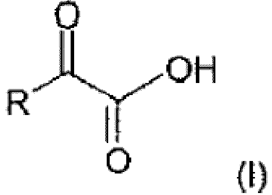 wobei in Formel (I):
R für ein Atom oder eine Gruppe steht, das bzw. die ausgewählt ist aus: i) Wasserstoff, ii) Carboxyl -C(O)OH, iii) geradkettigem oder verzweigtem C₁-C₆-Alkyl oder geradkettigem oder verzweigtem C₁-C₆-Alkyl, das durch mindestens einen Hydroxylrest -OH substituiert ist, oder geradkettigem oder verzweigtem C₁-C₆-Alkyl, das durch ein Halogen wie Br substituiert ist; iv) gegebenenfalls substituiertem Phenyl, v) gegebenenfalls substituiertem Benzyl, wobei iv) und v) vorzugsweise gegebenenfalls durch mindestens einen -OH- oder -C(O)OH-Rest substituiert sind; vi) einem Indolylrest und vii) einem Imidazolylmethylrest und Tautomeren davon wie 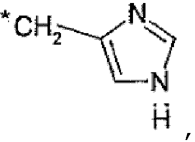 wobei * für den mit dem Rest des Moleküls verknüpften Teil steht, wobei es sich bei "Derivaten" der Dicarbonylverbindungen der Formel (I) um Ester der Carboxylgruppe(n), Amide der Carboxylgruppe(n) und (Thio)acetale und Hemi-(thio)acetale der Carbonylfunktion(en) der Verbindungen der Formel (I) in freier Form oder gegebenenfalls in Form von Salzen oder Hydraten handelt,
vorzugsweise eine oder mehrere Dicarbonylverbindung(en) nach einem der Ansprüche 3 bis 6,
und ii) mindestens ein Aminosilikon, vorzugsweise mindestens ein Aminosilikon nach einem der Ansprüche 7 bis 10, wobei die Zusammensetzung einen pH-Wert kleiner oder gleich 4 aufweist.

## Revendications

1. Procédé de lissage de fibres kératiniques telles que les cheveux, comprenant l'application sur lesdites fibres d'une composition cosmétique comprenant :
i) un ou plusieurs composés dicarbonylés répondant à la formule (I) ci-après et/ou leurs dérivés et/ou leurs hydrates et/ou leurs sels: 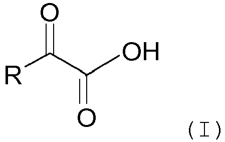 formule (I) dans laquelle :
**R** représente un atome ou groupement choisi parmi i) hydrogène, ii) carboxyle -C(O)OH, iii) C₁-C₆-alkyle linéaire ou ramifié qui est éventuellement substitué, de préférence par au moins un radical hydroxyle -OH, un radical carboxyle -C(O)-OH ou halogène tel que Br ; iv) phényle éventuellement substitué, v) benzyle éventuellement substitué, iv) et v) étant de préférence éventuellement substitués par au moins un radical -OH ou -C(O)OH ; vi) un radical indolyle et vii) un radical imidazolylméthyle et ses tautomères, tel que : 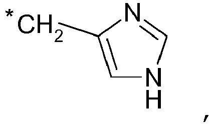 * représentant la partie reliée au reste de la molécule, les « dérivés » des composés dicarbonylés de formule (I) étant des esters d'un ou plusieurs groupements carboxyle, des amides d'un ou plusieurs groupements carboxyle, et des (thio)acétals et hémi(thio)acétals de la ou des fonctions carbonyle des composés de formule (I), sous forme libre ou éventuellement sous la forme de sels ou d'hydrates,
et
ii) au moins une silicone aminée,
la composition présentant un pH inférieur ou égal à 4, suivie d'un étape de lissage au moyen d'un fer de lissage.

2. Procédé selon la revendication 1, dans lequel le ou les composés dicarbonylés répondent à la formule **(I), R** représentant i) un atome d'hydrogène ou ii) un groupement C₁-C₆-alkyle linéaire ou ramifié éventuellement substitué par un groupement carboxyle.

3. Procédé selon l'une ou l'autre parmi les revendications précédentes, dans lequel le ou les composés dicarbonylés de formule **(I)** et/ou leurs dérivés selon la revendication 1 et/ou leurs hydrates et/ou leurs sels, sont choisis parmi l'acide glyoxylique et l'acide pyruvique, l'un de leurs dérivés, leurs sels et leurs hydrates, de préférence parmi l'acide glyoxylique, l'un de ses dérivés selon la revendication 1 et les formes d'hydrate de ces composés.

4. Procédé selon l'une quelconque des revendications précédentes, dans lequel le ou les dérivés dicarbonylés de formule **(I)** et/ou leurs dérivés selon la revendication 1 sont choisis parmi les esters d'acide glyoxylique, les amides d'acide glyoxylique, les (thio)acétals et hémi(thio)acétals d'acide glyoxylique, et les (thio)acétals et hémi(thio)acétals d'ester d'acide glyoxylique.

5. Procédé selon la revendication 3, dans lequel l'acide glyoxylique se présente sous sa forme d'hydrate.

6. Procédé selon l'une quelconque des revendications précédentes, dans lequel la composition comprend de 0,1% à 20% en poids d'un ou de plusieurs composés dicarbonylés répondant à la formule **(I)** et/ou leurs dérivés selon la revendication 1 et/ou leurs hydrates et/ou leurs sels, préférablement de 3% à 10% en poids par rapport au poids total de la composition.

7. Procédé selon l'une quelconque des revendications précédentes, dans lequel la ou les silicones aminées sont choisies parmi les silicones ***(a)*** à ***(e)*** ci-après :
***(a)*** les composés répondant à la formule **(Ia)** ci-après :
(R₁)ₐ(T)₃₋ₐ-Si[OSi(T)₂]ₙ-[OSi(T)₂]ₙ-(R₁)_{2-b}]ₘ-OSi(T)₃₋ₐ(R₁)ₐ **(Ia)**
formule **(Ia)** dans laquelle :
**T** représente un atome d'hydrogène, ou un groupement phényle, hydroxyle (-OH), ou C₁-C₈-alkyle, et de préférence méthyle, ou C₁-C₈-alcoxy, de préférence méthoxy ;
**a** désigne le nombre 0 ou un nombre entier allant de 1 à 3, et de préférence 0 ;
**b** désigne 0 ou 1, et en particulier 1 ;
**m** et **n** sont des nombres entiers tels que la somme (n + m) peut varier notamment de 1 à 2000 et en particulier de 50 à 150, n pouvant désigner un nombre allant de 0 à 1999 et notamment de 49 à 149 et m pouvant désigner un nombre allant de 1 à 2000, et notamment de 1 à 10 ;
**R₁** est un groupement monovalent de formule -C_{q}H_{2q}L où **q** est un nombre entier allant de 2 à 8 et L est un groupement aminé éventuellement quaternisé choisi parmi les groupements :
-N(R₂)-CH₂-CH₂-N(R₂)₂,
-N(R₂)₂,
-N⁺(R₂)₃Q⁻,
-N⁺(R₂)(H)₂Q⁻,
-N⁺(R₂)₂HQ⁻,
-N(R₂)-CH₂-CH₂-N⁺(R₂)(H)₂Q⁻ ;
où R₂ peut désigner un atome d'hydrogène, un groupement phényle, un groupement benzyle, ou un groupement hydrocarboné saturé monovalent, par exemple un groupement C₁-C₂₀-alkyle, et Q⁻ représente un contre-ion anionique tel qu'un ion halogénure, par exemple fluorure, chlorure, bromure ou iodure ;
***(b)*** les composés répondant à la formule **(Ib)** ci-après : 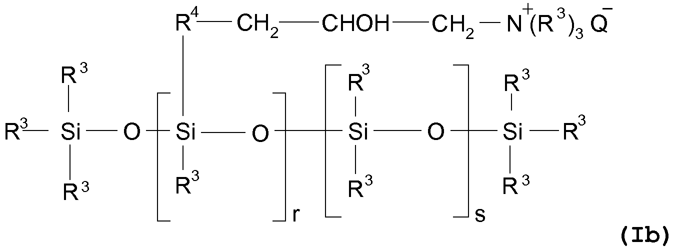 formule **(Ib)** dans laquelle :
**R³** représente un groupement hydrocarboné monovalent en C₁-C₁₈, et en particulier un groupement C₁-C₁₈-alkyle ou C₂-C₁₈-alcényle, par exemple méthyle ;
**R⁴** représente un groupement hydrocarboné divalent, notamment un groupement C₁-C₁₈-alkylène ou un groupement divalent C₁-C₁₈-alkylèneoxy, par exemple en C₁-C₈ ;
**Q⁻** représente un contre-ion anionique tel que choisi parmi les ions halogénure, notamment chlorure ;
**r** représente une valeur statistique moyenne allant de 2 à 20 et en particulier de 2 à 8 ;
**s** représente une valeur statistique moyenne comprise inclusivement entre 20 et 200 et en particulier comprise inclusivement entre 20 et 50 ;
***(c)*** les silicones d'ammonium quaternaire, notamment de formule **(Ic)** : 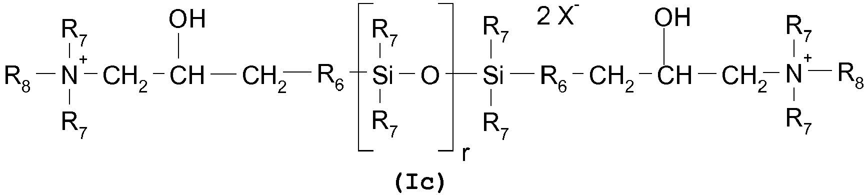 formule **(Ic)** dans laquelle :
**R₆** représente un groupement hydrocarboné divalent, notamment un groupement C₁-C₁₈-alkylène ou un groupement divalent C₁-C₁₈-alkylèneoxy, par exemple en C₁-C₈ relié à l'atome de Si par une liaison Si-C ;
**R₇,** qui peuvent être identiques ou différents, représentent un groupement hydrocarboné monovalent contenant de 1 à 18 atomes de carbone, et en particulier un groupement C₁-C₁₈-alkyle, un groupement C₂-C₁₈-alcényle ou un cycle comprenant 5 ou 6 atomes de carbone, par exemple méthyle ;
**R₈**, qui peuvent être identiques ou différents, représentent chacun un atome d'hydrogène, un groupement hydrocarboné monovalent contenant de 1 à 18 atomes de carbone, et en particulier un groupement C₁-C₁₈-alkyle, un groupement C₂-C₁₈-alcényle, ou un groupement -R₆-N(H)-C(O)-R₇, R₆ et R₇ étant tels que définis précédemment ;
**X**⁻**,** qui peut être identique ou différent, représente un contre-ion anionique tel qu'un ion halogénure, notamment chlorure ou un contre-ion anionique dérivé d'un acide organique tel que carboxylate de (C₁-C₆)alkyle ;
**r** représente une valeur statistique moyenne comprise inclusivement entre 2 et 200 et en particulier comprise entre 5 et 100 ;
**(d)** les silicones aminées de formule **(Id)** : 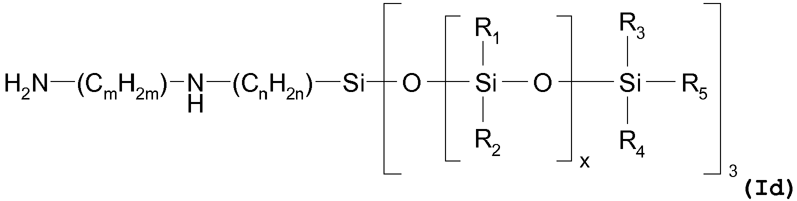 formule **(Id)** dans laquelle :
**R₁, R₂, R₃** et **R₄,** qui peuvent être identiques ou différents, désignent chacun un groupement C₁-C₄-alkyle ou un groupement aryle tel que phényle,
**R₅** désigne un groupement C₁-C₄-alkyle ou un groupement hydroxyle,
**n** et **m,** qui peuvent être identiques ou différents, représentent un nombre entier compris inclusivement entre 1 et 5, et
**x** est tel que l'indice d'amine soit compris entre 0,01 et 1 méq/g ;
**(e)** les silicones aminées à groupements polyalcoxylène de formule **(Ie)** : 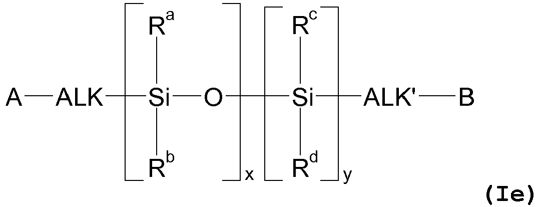 formule **(Ie)** dans laquelle :
**R^{a}, R^{b}, R^{c},** et **R^{d},** qui peuvent être identiques ou différents, représentent un groupement hydroxy, ou (C₁-C₁₀)alkyle linéaire ou ramifié, de préférence **R^{a}, R^{b}, R^{c},** et **R^{d}** représentent un groupement (C₁-C₆) alkyle, qui est plus particulièrement linéaire, tel que méthyle ;
**ALK** et **ALK',** qui peuvent être identiques ou différents, représentent un groupement (C₁-C₁₀)alkylène linéaire ou ramifié, qui est de préférence linéaire, tel que propylène ;
**A** et **B,** qui peuvent être identiques ou différents, représentent le groupement aminopolyalcoxy ci-après :
R^{e}R^{f}N-[ALK"'-O]_{z}-[ALK"'-O]_{w}-ALKₐ-N(R^{g})-[ALK_{b}-O]_{q}-*
où :
* représente le point de fixation du radical au reste de la molécule via ALK ou ALK' ;
**R^{e}, R^{f}** et **R^{g},** qui peuvent être identiques ou différents, représentent un atome d'hydrogène, ou un groupement (C₁-C₁₀)alkyle linéaire ou ramifié, et de préférence **R^{e}, R^{f}** et **R^{g}** représentent un atome d'hydrogène ;
**ALK"** et **ALK"',** qui peuvent être identiques ou différents, représentent un groupement (C₁-C₁₀)alkylène linéaire ou ramifié, de préférence en C₂ ou en C₃ ; plus particulièrement, ALK" représente un groupement divalent -CH₂-CH(CH₃)- et ALK"' représente un groupement éthylène ;
**ALKₐ** et **ALK_{b},** qui peuvent être identiques ou différents, représentent un groupement (C₁-C₁₀)alkylène linéaire ou ramifié, qui est éventuellement substitué de préférence par un groupement hydroxyle, et qui est de préférence en C₂ ou en C₃ ; plus particulièrement ALKₐ représente un groupement éthylène ou propylène ou un groupement divalent -CH₂-CH(CH₃)- et ALK_{b} représente un groupement divalent -CH₂-CH(OH)-CH₂-;
**q,** qui peut être identique ou différent, représente 0 ou 1, de préférence 1 ;
**w,** qui peut être identique ou différent, représente un nombre entier, de préférence la somme des valeurs de w (w de A + w de B) ayant une valeur moyenne comprise inclusivement entre 10 et 100, plus particulièrement comprise inclusivement entre 20 et 60, et plus préférablement entre 30 et 50, tel que 40-41 ;
**z,** qui peut être identique ou différent, représente un nombre entier, de préférence la somme des valeurs de z (z de A + z de B) ayant une valeur moyenne comprise inclusivement entre 1 et 20, plus particulièrement comprise inclusivement entre 1 et 10, et plus préférablement entre 2 et 5, tel que 3 ;
***(I'e)*** les silicones aminées à groupements polyalcoxylène constituées de bloc(s) de polysiloxane et de bloc(s) de polyalcoxylène comprenant au moins un groupement amine, en particulier :
- celles de formule **(I'e)** : 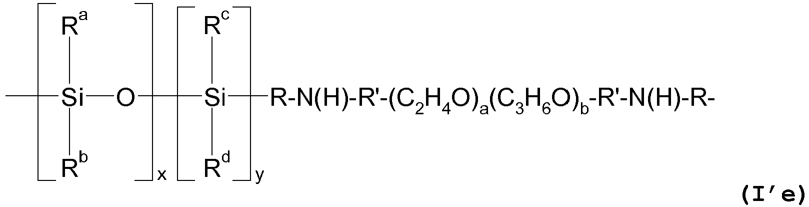 formule **(I'e)** dans laquelle :
**R^{a}, R^{b}, R^{c},** et **R^{d},** qui peuvent être identiques ou différents, représentent un groupement hydroxyle ou (C₁-C₁₀)alkyle linéaire ou ramifié, et de préférence **R^{a}**, **R^{b}**, **R^{c}**, et **R^{d}** représentent un groupement (C₁-C₄) alkyle, qui est plus particulièrement linéaire, tel que méthyle ;
**R** et **R"**, qui peuvent être identiques ou différents, représentent un radical C₂-C₆-alkylène linéaire ou ramifié, éventuellement hydroxylé, et éventuellement interrompu par un atome d'oxygène ;
**a** et **b,** qui peuvent être identiques ou différents, représentent un nombre allant de 0 à 100 ;
**R'** et **R'''**, qui peuvent être identiques ou différents, représentent un atome d'hydrogène ou un radical C₁-C₄-alkyle tel qu'un radical méthyle ;
**x** désigne un nombre entier allant de 1 à 500 et **y** désigne un nombre entier allant de 1 à 10 ;
- celles contenant des motifs de formule **(IIe)** 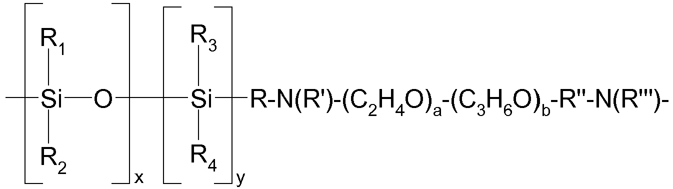 formule **(IIe)** dans laquelle,
**R₁** à **R₄**, qui peuvent être identiques ou différents, représentent un radical C₁-C₄-alkyle, de préférence méthyle ;
**R** et **R"**, qui peuvent être identiques ou différents, représentent un radical C₂-C₆-alkylène linéaire ou ramifié, éventuellement hydroxylé, et éventuellement interrompu par un atome d'oxygène ;
**a** et **b,** qui peuvent être identiques ou différents, représentent un nombre entier allant de 0 à 100 ;
**R'** et **R'''**, qui peuvent être identiques ou différents, représentent un atome d'hydrogène ou un radical C₁-C₄-alkyle tel qu'un radical méthyle ; et
**x** désigne un nombre allant de 1 à 500 et **y** un nombre allant de 1 à 10.

8. Procédé selon l'une quelconque des revendications précédentes, dans lequel la ou les silicones aminées sont choisies parmi les silicones aminées à groupements polyalcoxylène constituées de bloc(s) de polysiloxane et de bloc(s) de polyalcoxylène comportant au moins un groupement amine, en particulier celles de formule **(Ie)** ou **(Ie')** telles que définies selon la revendication précédente, et particulièrement la ou les silicones aminées sont choisies parmi :
i) les silicones aminées de type bisamino-polyéthylène glycol/polypropylène glycol-41-3 aminoéthyl PG(glycidopropyl)-propyl diméthicone, en particulier de formule **(IIe)** suivante : 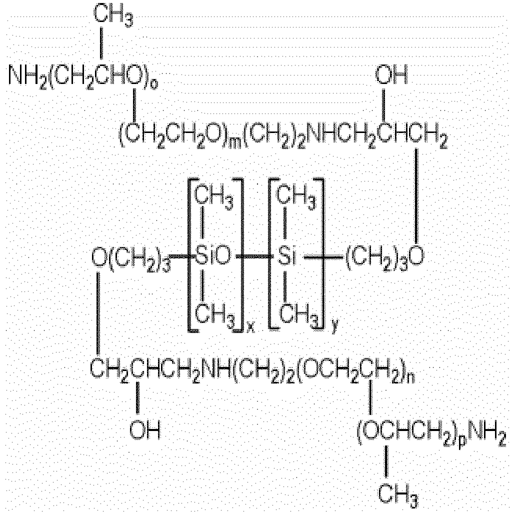 formule (IIe) dans laquelle m et n, qui peuvent être identiques ou différents, représentent un nombre entier, la somme m + n ayant une valeur moyenne de 41 et o et p qui peuvent être identiques ou différents, représentent un nombre entier, la somme o + p ayant une valeur moyenne de 3.

9. Procédé selon l'une quelconque des revendications précédentes, dans lequel la ou les silicones aminées sont choisies parmi les silicones aminées dérivées de la réaction entre un ou plusieurs copolymères de PEG-40/PPG-8 terminés par un groupement 2-aminopropyle et de bis-glycidoxypropyl diméthicone.

10. Procédé selon l'une quelconque des revendications précédentes, dans lequel la ou les silicones aminées sont présentes selon une quantité allant de 0,01% à 10%, mieux encore de 0,1% à 5% en poids, préférablement de 0,5% à 3% en poids, et encore plus préférablement de 0,5% à 1,5% en poids, par rapport au poids total de la composition.

11. Procédé selon l'une quelconque des revendications précédentes, dans lequel le rapport pondéral entre la quantité de silicone(s) aminée(s) telle(s) que définie(s) selon les revendications 1 et 7 à 9, d'une part, et la quantité de composés dicarbonylés répondant à la formule (I) et/ou leurs dérivés selon la revendication 1 et/ou leurs hydrates et/ou leurs sels tels que définis selon les revendications 1 à 6, va de 0,01 à 100, encore plus préférablement de 0,01 à 20, mieux encore de 0,05 à 10, et toujours mieux de 0,05 à 1.

12. Procédé selon l'une quelconque des revendications précédentes, **caractérisé en ce qu'**il est aqueux et comprend de l'eau selon une concentration allant de préférence de 5% à 98%, mieux encore de 5% à 90%, et toujours mieux de 10% à 90% en poids par rapport au poids total de la composition.

13. Procédé selon l'une quelconque des revendications précédentes, **caractérisé en ce que** la composition résulte du mélange de plusieurs compositions.

14. Procédé selon l'une quelconque des revendications précédentes, dans lequel le temps de contact de la composition avec les cheveux est compris entre 10 et 60 minutes.

15. Procédé selon l'une quelconque des revendications précédentes, dans lequel les cheveux sont lissés au moyen d'un fer de lissage à une température d'au moins 150°C, et de préférence comprise entre 150 et 250°C.

16. Utilisation d'une composition comprenant :
i) un ou plusieurs composés dicarbonylés répondant à la formule (I) ci-après et/ou leurs dérivés et/ou leurs hydrates et/ou leurs sels: 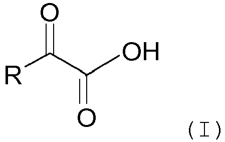 formule **(I)** dans laquelle :
**R** représente un atome ou groupement choisi parmi i) hydrogène, ii) carboxyle -C(O)OH, iii) C₁-C₆-alkyle linéaire ou ramifié qui est éventuellement substitué, de préférence par au moins un radical hydroxyle -OH, un radical carboxyle -C(O)-OH ou halogène tel que Br ; iv) phényle éventuellement substitué, v) benzyle éventuellement substitué, iv) et v) étant de préférence éventuellement substitués par au moins un radical -OH ou -C(O)OH ; vi) un radical indolyle et vii) un radical imidazolylméthyle et ses tautomères, tel que : 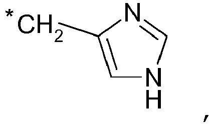 * représentant la partie reliée au reste de la molécule,
les « dérivés » des composés dicarbonylés de formule (I) étant des esters d'un ou plusieurs groupements carboxyle, des amides d'un ou plusieurs groupements carboxyle, et des (thio)acétals et hémi(thio)acétals de la ou des fonctions carbonyle des composés de formule (I), sous forme libre ou éventuellement sous la forme de sels ou d'hydrates, et
ii) au moins une silicone aminée,
la composition présentant un pH inférieur ou égal à 4, pour le lissage/défrisage des fibres kératiniques, notamment des cheveux.

17. Composition cosmétique, en particulier une composition pour les cheveux, comprenant
i) un ou plusieurs composés dicarbonylés répondant à la formule (I) ci-après et/ou leurs dérivés et/ou leurs hydrates et/ou leurs sels: 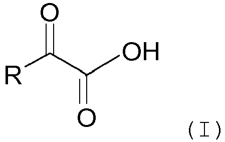 formule **(I)** dans laquelle :
**R** représente un atome ou groupement choisi parmi i) hydrogène, ii) carboxyle -C(O)OH, iii) C₁-C₆-alkyle linéaire ou ramifié ou C₁-C₆-alkyle linéaire ou ramifié substitué par au moins un radical hydroxyle -OH ou C₁-C₆-alkyle linéaire ou ramifié substitué par halogène tel que Br ; iv) phényle éventuellement substitué, v) benzyle éventuellement substitué, iv) et v) étant de préférence éventuellement substitués par au moins un radical -OH ou -C(O)OH ; vi) un radical indolyle et vii) un radical imidazolylméthyle et ses tautomères, tel que : 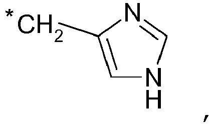 * représentant la partie reliée au reste de la molécule,
les « dérivés » des composés dicarbonylés de formule (I) étant des esters d'un ou plusieurs groupements carboxyle, des amides d'un ou plusieurs groupements carboxyle, et des (thio)acétals et hémi(thio)acétals de la ou des fonctions carbonyle des composés de formule (I), sous forme libre ou éventuellement sous la forme de sels ou d'hydrates,
préférablement un ou plusieurs composés dicarbonylés selon l'une quelconque des revendications 3 à 6, et
ii) au moins une silicone aminée, préférablement au moins une silicone aminée selon l'une quelconque des revendications 7 à 10, la composition présentant un pH inférieur ou égal à 4.
